(19) Europäisches Patentamt / European Patent Office / Office européen des brevets

(11) **EP 4 572 839 B1**

(12) **EUROPEAN PATENT SPECIFICATION**

(45) Date of publication and mention of the grant of the patent:
**01.04.2026 Bulletin 2026/14**

(21) Application number: **23768400.6**

(22) Date of filing: **16.08.2023**

(51) International Patent Classification (IPC):
**A61N 1/36** (2006.01)

(52) Cooperative Patent Classification (CPC):
**A61N 1/36071; A61N 1/36062; A61N 1/36139**

(86) International application number:
**PCT/US2023/030335**

(87) International publication number:
**WO 2024/039715 (22.02.2024 Gazette 2024/08)**

(54) **PAIN THERAPY OPTIMIZATION USING A MOBILITY METRIC**

SCHMERZTHERAPIEOPTIMIERUNG UNTER VERWENDUNG EINER MOBILITÄTSMETRIK

OPTIMISATION DE THÉRAPIE CONTRE LA DOULEUR AU MOYEN D'UNE MÉTRIQUE DE MOBILITÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.08.2022 US 202263398288 P**

(43) Date of publication of application:
**25.06.2025 Bulletin 2025/26**

(73) Proprietor: **Boston Scientific Neuromodulation Corporation**
**Valencia, CA 91355 (US)**

(72) Inventors:
• **SRIVASTAVA, Kyle Harish**
**Saint Paul, Minnesota 55105 (US)**
• **HERSHEY, Bradley Lawrence**
**Carrollton, Texas 75006 (US)**
• **HUYNH, Dat Thanh**
**North Hollywood, California 91601 (US)**
• **BAINS, Amarpreet Singh**
**Woodbury, Minnesota 55129 (US)**
• **MCDONALD, Matthew Lee**
**Glendale, California 91208 (US)**
• **HAHN, Benjamin Phillip**
**Austin, Texas 78730 (US)**
• **LECHLEITER, Kristen Marie**
**Davidson, North Carolina 28036 (US)**

(74) Representative: **Vossius & Partner**
**Patentanwälte Rechtsanwälte mbB**
**Siebertstrasse 3**
**81675 München (DE)**

(56) References cited:
**US-A1- 2021 060 343    US-B2- 10 631 777**
**US-B2- 11 089 997**

EP 4 572 839 B1

**Description**

TECHNICAL FIELD

This document relates generally to medical systems, and more particularly to systems and devices for pain management using patient mobility information.

BACKGROUND

Pain is one of the most common and among the most personally compelling reasons for seeking medical attention, and consumes considerable healthcare resources each year. The relation between etiology, underlying mechanisms and the specific symptoms and signs related to painful disorders is complex. Pain in an individual patient may be produced by more than one mechanism.

Chronic pain, such as pain present most of the time for a period of six months or longer during the prior year, is a highly pervasive complaint and consistently associated with psychological illness. Chronic pain may originate with a trauma, injury or infection, or there may be an ongoing cause of pain. Chronic pain may also present in the absence of any past injury or evidence of body damage. Common chronic pain can include headache, low back pain, cancer pain, arthritis pain, neurogenic pain (pain resulting from damage to the peripheral nerves or to the central nervous system), or psychogenic pain (pain not due to past disease or injury or any visible sign of damage inside or outside the nervous system).

Chronic pain may be treated or alleviated using medications, acupuncture, surgery, and neuromodulation therapy such as local electrical stimulation or brain stimulation, among others. Examples of neuromodulation include Spinal Cord Stimulation (SCS), Deep Brain Stimulation (DBS), Peripheral Nerve Stimulation (PNS), and Functional Electrical Stimulation (FES). Implantable neuromodulation systems have been applied to deliver such a therapy. An implantable neuromodulation system may include an implantable neurostimulator, also referred to as an implantable pulse generator (IPG), which can electrically stimulate tissue or nerve centers to treat nervous or muscular disorders. In an example, an IPG can deliver electrical pulses to a specific region in a patient's spinal cord, such as particular spinal nerve roots or nerve bundles, to create an analgesic effect that masks pain sensation. US 10 631 777 B2 is related art which discloses a system which includes a motion sensor configured to sense at least one functional signal indicative a physical state of the subject. The at least one functional signal may include at least one motor activity signal or at least one sleep state signal. A pain analyzer circuit may extract, from the functional signal, signal metrics indicative of subject motor control or kinetics, and generate a pain score using the signal metrics. The pain score may be output to a user or a process. The system may additionally include an electrostimulator to generate and deliver a closed-loop pain therapy according to the pain score.

SUMMARY

Patients with chronic pain usually have certain levels of mobility limitations or difficulty with daily tasks requiring adequate physical functions. Monitoring mobility can help assess chronic pain and efficacy of pain therapies such as neuromodulation therapy. This document discusses systems, devices, and methods for monitoring and managing patients with chronic pain. According to one example, a patient monitoring system comprises a sensor circuit to sense a physiological or functional signal indicative of or correlated to patient mobility, an electrostimulator to generate and deliver neuromodulation therapy to the patient, and a controller circuit to generate a mobility metric using the sensed physiological or functional signal. The mobility metric represents respective times spent in different activity intensity levels associated with one or more types of activities during a time period (e.g., a day). The controller circuit can trend the mobility metric over time and determine a progress toward a personalized mobility goal of the patient, and present the trended mobility metric or the progress on a user interface. In some examples, the physiological or functional signal can be sensed in response to a neuromodulation therapy for managing chronic pain, and the controller circuit can adjust the neuromodulation therapy based on the trended mobility metric or the progress toward the mobility goal. The invention is defined by the independent claim 1. Further aspects of the invention are defined by the dependent claims.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.

FIG. 1 illustrates generally an example of a patient monitoring and pain management system and portions of an environment in which the system may operate.

FIG. 2 illustrates generally an example of a Spinal Cord Stimulation (SCS) system.

FIG. 3 illustrates an example of a mobility monitor system configured to monitor and analyze patient mobility.

FIGS. 4A-4C illustrate examples of mobility metric including mobility time distribution and mobility scores.

FIG. 5A illustrates an example of activity type- or activity context-based mobility metric.

FIG. 5B illustrates an example of mobility metric respectively evaluated for each of a number of different stimulation settings or stimulation programs.

FIG. 5C illustrates an example of mobility metric respectively evaluated for each of a number of different stimulation settings or stimulation programs when the patient engages the same type of activity or under the same activity context.

FIG. 6A illustrates a graphic representation of a change from a previous mobility distribution.

FIG. 6B illustrates an graphical comparison between a mobility distribution and a target mobility distribution of a personalized mobility goal across a plurality of activity intensity bins.

FIG. 7 illustrates a mobility score trend, a physical function score trend, and a QoL score trend of the same patient during a specific time period.

FIG. 8 illustrates an example of a non-claimed method for monitoring and analyzing patient mobility.

FIG. 9 illustrates generally a block diagram of an example machine upon which any one or more of the techniques (e.g., methodologies) discussed herein may perform.

DETAILED DESCRIPTION

By way of example, chronic pain management may involve monitoring patient pain symptoms and physical, functional, and emotional well-being, determining appropriate treatment options (e.g., neuromodulation therapies with an implantable device), and evaluating patient response to pain therapy. Effective monitoring and accurate pain assessment are important for managing patients with chronic pain. Such assessment may include a pain rating, such as on a numerical scale of 1 to 10 (with 10 being the worst pain) or similar descriptors of pain intensities. Such pain rating, however, may have several drawbacks. For one thing, chronic pain patients may have different tolerances to pain, making the pain ratings subjective and less comparable among patients. Additionally, the pain rating alone may not reflect patient physical and functional capacities. Although chronic pain may limit patient functional capacity and cause mobility issues, an improvement in pain sensation (e.g., a lower pain rating) may not always be accompanied by or synchronized with a progress in the patient's functional capacities, mobility status, and overall quality of life. For example, a chronic pain patient may report a sizable reduction in pain yet remains to be bedridden without gaining improvement in his/her physical or functional capacities. In another example, a chronic pain patient may not report substantial pain reduction, even though he/she has started walking, sleeping normally, and engaging in more activities.

Functional assessment has been performed clinically to optimize function-based strategy for treating chronic pain. A function-based strategy involves measuring a patient's progress not in pain relief (e.g., a decrease in pain rating), but in physical functions such as sleeping, walking, working, connecting with friends, or other behavioral and social activities in daily life. Such functional assessment can provide more objective insight into patient physical and social capacities and quality of life (QoL), and can be used to evaluation an efficacy of a pain therapy (e.g., neuromodulation therapy such as SCS) received by the patient or whether the pain therapy needs to be adjusted.

For patients with an implantable neuromodulation device for pain control, assessment of pain and mobility or functional capacities are typically performed in a clinic or other medical facilities. For some patients and on some occasions, such in-person assessment in a clinical setting may not always be feasible or practical. For example, some patients with chronic pain and under long-term treatment (e.g., via an implantable neuromodulator) may experience gradual physiological, functional, or emotional changes, which can get unnoticed for an extended period of time when the patient is outside a clinical setting until it becomes symptomatic and get managed during a clinic visit. Continuous or periodic monitoring of such patients would be desirable to timely detect pain progression and changes in patient functional abilities. On the other hand, some chronic-pain patients may experience sudden change in their pain sensation, and require immediate pain assessment, a therapy titration, or neuromodulation device check. However, geographic barriers to medical facilities and resources may prevent or delay the clinical evaluation and treatment titration.

The present inventors have recognized that a remotely-accessible patient monitoring and pain management platform can be more desirable and advantageous in certain situations than a conventional in-person visit at a clinic. Such a patient monitoring and pain management platform can monitor patient mobility continuously, periodically, or at any desired time between clinic visits. Disclosed herein are systems, devices, and methods for monitoring and managing patients with chronic pain. A patient monitoring system comprises a sensor circuit to sense a physiological or functional signal indicative of or correlated to patient mobility, and a controller circuit to generate a mobility metric using the sensed signal. The mobility metric represents respective times spent in different activity intensity levels in association with one or more types of activities during a time period. The controller circuit can trend the mobility metric over time and determine a progress toward a personalized mobility goal, and present the trended mobility metric or the progress to a user. In some examples, the physiological or functional signal can be sensed responsive to a neuromodulation therapy for managing chronic pain, and the controller circuit can adjust the neuromodulation therapy based on the trended mobility metric or the progress toward the mobility goal.

The patient monitoring and pain management system and methods of using the same, as described in this document, may improve the technology of device-based pain management. According to one aspect of the present subject matter, mobility metrics may be evaluated when a patient is engaging in daily activities such as grocery shopping, walking a dog, doing yardwork, working out in gym, cooking, among other activities. Such activity type or a context information may be "tagged" with the mobility metrics to produce activity type- or context-based mobility metrics, which can help improve interpretation of changes in mobility and optimize neuromodulation therapy when the patient engages in a particular activity. According to some embodiments, the mobility metric may be correlated to patient-reported physical functions, or used to generate a quality of life (QoL) score. The correlation and the QoL score enable more comprehensive and objective assessment of patient overall physical health and/or response to neuromodulation therapy. According to some embodiments, the mobility metric may be tracked against a personalized mobility goal of the patient. The mobility goal may be set based on patient baseline mobility characteristics, or population-based mobility data from patients with medical conditions or demographics similar to the patient being evaluated (the "similar" patients). The mobility goal thus established is not only tailored for individual patient health status, but also enables assessing patient improvement in mobility capacity relative to the group of "similar" patients. According to some embodiments, the mobility metric may be estimated by applying signals acquired by a variety of physiological sensors to respective trained mobility estimation models. Each model may be calibrated against a "standard" mobility metric determined using signals sensed by an accelerometer, such that the estimated mobility metric may be comparable to accelerometer-based mobility metric within a specified tolerance level. As the variety of physiological sensors may be included in different devices (e.g., an implantable medical device, a portable or wearable device such as a smartphone, a smart watch, etc.), the mobility estimation model enables "plug-and-play" capability of estimating a mobility metric from a range of existing devices with high estimation fidelity, yet without requiring device- or sensor-specific mobility algorithms. This can improve mobility assessment efficiency and reduce overall development and operation cost.

The patient monitoring and pain management system described herein can be implemented using a combination of hardware and software designed to provide a pain management regimen to increase therapeutic efficacy, increase patient satisfaction for neurostimulation therapies, reduce side effects, and/or increase device longevity. The present system may be applied in any neurostimulation (neuromodulation) therapies, including but not limited to SCS, DBS, PNS, FES, and Vagus Nerve Stimulation (VNS) therapies. In various examples, instead of providing closed-loop pain therapies, the systems, devices, and methods described herein may be used to monitor the patient and assess pain that either occurs intrinsically or is induced by nerve block procedures or radiofrequency ablation therapies, among others. The remote patient monitoring and pain management system may provide recommendations to the patient or a clinician regarding pain treatment.

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the spirit and scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

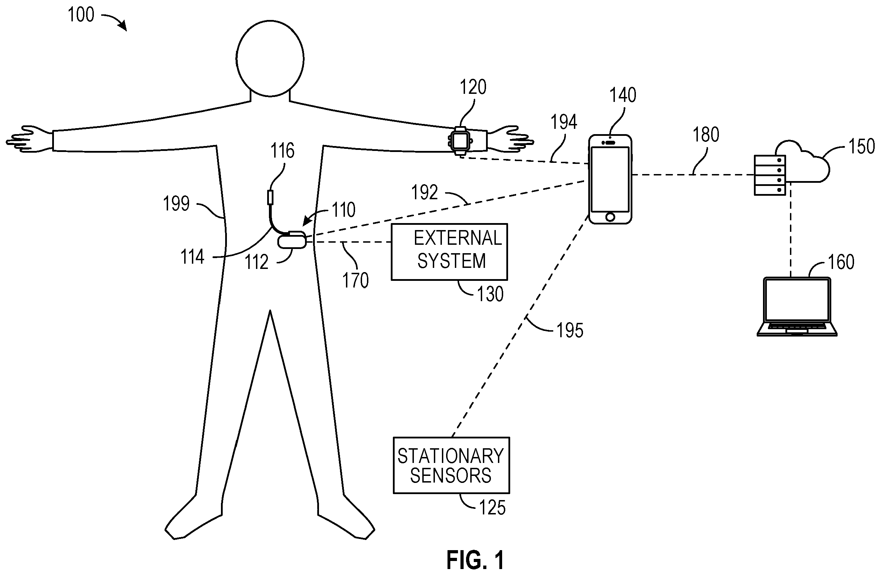

FIG. 1 illustrates, by way of example and not limitation, an example of a patient monitoring and pain management system 100 and portions of an environment in which the system 100 may operate. The system 100 may be used to monitor a patient having an ambulatory device, such as an implantable neuromodulator for managing chronic pain. In an example, the implantable neuromodulator can be a neurostimulator configured to deliver stimulation pulses or other energy modalities to modulate one or more neural targets, such as spinal cord, for controlling pain or other neurological or autonomic conditions.

The patient monitoring and pain management system 100 may include one or more of an implantable system 110 for implantation in the body 199 of the patient, a wearable electronic device 120 to be worn on the patient body 199 (such as on a wrist or an arm as shown), an external system 130, a mobile device 140 operable by a patient, a cloud-computing device 150, and one or more user terminals 160. The implantable system 110 may include an ambulatory medical device (AMD), such as an implantable pulse generator (IPG) 112, a lead system 114, and one or more electrodes 116. The IPG 112 may be configured for subcutaneous implant in a patient's chest, abdomen, or other parts of the body 199. The IPG 112 may include a hermetically sealed housing that houses sensing circuitry to sense physiological or functional signals from the patient via sensing electrodes or ambulatory sensors associated with the patient and in communication with the IPG 112. In some examples, the sensing electrodes or the ambulatory sensors may be included within the IPG 112. Physiological or functional signals may be sensed during a pain episode. The sensed physiological or functional signals may include at least one signal indicative of muscle electrical or mechanical activity at a specific body location. The IPG 112 may generate signal metrics indicative of muscle tension from the sensed muscle electrical or mechanical activity signal, and characterize and quantify the pain, such as to determine onset, intensity, severity, duration, or patterns of the pain experienced by the subject. The IPG 112 may generate an alert to indicate occurrence of a pain episode, pain exacerbation, or efficacy of pain therapy, and present the alert to a clinician.

The IPG 112 may provide neuromodulation therapy to the patient, such as therapies for treating or alleviating pain. The IPG 112 may generate electrical, magnetic, or other types of energy. In some examples, in addition or alternative to the IPG 112, a drug delivery system such as a drug infusion pump can be included to deliver pain medication to the patient, such as morphine sulfate or ziconotide, among others.

The IPG 112 may include electrostimulation circuitry that generates electrostimulation pulses to stimulate a neural target via the electrodes 116 operably connected to the IPG 112. In an example, the electrodes 116 may be positioned on or near a port of the spinal cord of the patient, and the electrostimulation circuitry may be configured to deliver SCS to alter or alleviate pain. In another example, the electrodes 116 may be surgically placed at other neural targets such as a brain or a peripheral neutral tissue, and the electrostimulation circuitry may be configured to deliver brain or peripheral stimulations. Examples of electrostimulation may include deep brain stimulation (DBS), trigeminal nerve stimulation, occipital nerve stimulation, vagus nerve stimulation (VNS), sacral nerve stimulation, sphenopalatine ganglion stimulation, sympathetic nerve modulation, adrenal gland modulation, baroreceptor stimulation, or transcranial magnetic stimulation, spinal cord stimulation (SCS), dorsal root ganglia (DRG) stimulation, motor cortex stimulation (MCS), transcranial direct current stimulation (tDCS), transcutaneous spinal direct current stimulation (tsDCS), pudendal nerve stimulation, multifidus muscle stimulation, transcutaneous electrical nerve stimulation (TENS), tibial nerve stimulation, among other peripheral nerve or organ stimulation. The IPG 112 may additionally or alternatively provide therapies such as radiofrequency ablation (RFA), pulsed radiofrequency ablation, ultrasound therapy, high-intensity focused ultrasound (HIFU), optical stimulation, optogenetic therapy, magnetic stimulation, other peripheral tissue stimulation therapies, other peripheral tissue denervation therapies, or nerve blocks or injections.

In various examples, the electrodes 116 may be disposed on one or more leads of the lead system 114 electrically coupled to the IPG 112. In an example, the lead system 114 may include a directional lead that includes at least some segmented electrodes circumferentially disposed about the directional lead. Two or more segmented electrodes may be distributed along a circumference of the lead. The actual number and shape of leads and electrodes may vary according to the intended application. Detailed description of construction and method of manufacturing percutaneous stimulation leads are disclosed in U.S. patent No. 8,019,439, entitled "Lead Assembly and Method of Making Same," and U.S. Patent No. 7,650,184, entitled "Cylindrical Multi-Contact Electrode Lead for Neural Stimulation and Method of Making Same," the disclosures of which are incorporated herein by reference. The electrodes 116 may provide an electrically conductive contact providing for an electrical interface between the IPG 112 and tissue of the patient. The neurostimulation pulses are each delivered from the IPG 112 through a set of electrodes selected from the electrodes 116. In various examples, the neurostimulation pulses may include one or more individually defined pulses, and the set of electrodes may be individually definable by the user for each of the individually defined pulses.

Although the discussion herein with regard to pain management is focused on implantable devices such as the IPG 112, this is meant only by way of example and not limitation. The patient monitoring and pain management techniques discussed herein may also be applied to pain management via non-implantable devices, such as subcutaneous devices, wearable devices (e.g., wrist watch, patches, ring, necklace, garment- or shoe-mounted device, etc.), or other external medical devices, or a combination of implantable, wearable, or other external devices. The therapy, such as electrostimulation or medical therapies, may be used to treat various neurological disorders other than pain including, for example, epilepsy, obsessive compulsive disorder, tremor, Parkinson's disease, or dystonia, among other movement and affective disorders.

The external system 130 can be communicatively coupled to the IPG 112 via a communication link 170. The external system 130 may include a dedicated hardware/software system such as a programmer, a remote server-based patient management system, or alternatively a system defined predominantly by software running on a standard personal computer. The external system 130 may be configured to control the operation of the IPG 112, such as to program the IPG 112 for delivering neuromodulation therapy. The external system 130 may additionally receive via the communication link 170 information acquired by IPG 112, such as one or more sensor signals. In an example, a user may use the external system 130 to program the IPG 112 to deliver pain therapy in a closed-loop fashion based on the signals received from the IPG 112. The external system 130 may include a display that can display information including, for example, signal received by the IPG 112, a pain or paresthesia map indicating pain or paresthesia perception at various body locations of the patient, programming of pain therapy such as a stimulation setting including parameters and their values, among others. In some examples, the external system 130 may generate an alert notification. The alert notifications may include a Web page update, phone or pager call, E-mail, SMS, text or "Instant" message, as well as a message to the patient and a simultaneous direct notification to emergency services and to the clinician. Other alert notifications are possible.

The communication link 170 may include one or more communication channels and intermediate devices between the external system and the IPG 112. In an example, the communication link 170 may be a wired connection. In another example, the communication link 170 may be a wireless link, such as an inductive telemetry link, a capacitive telemetry link, a radiofrequency (RF) telemetry link, or wireless telemetry based on, for example, "strong" Bluetooth or IEEE 802.11 wireless fidelity "WiFi" interfacing standards. Other configurations and combinations of patient data source interfacing are possible. In an example where the external system 130 includes a remote server such as configured as a uni-, multi- or distributed computing and processing system, the communication link 170 may be based on the Transmission Control Protocol/Internet Protocol (TCP/IP) network communication specification, although other types or combinations of networking implementations are possible. Similarly, other network topologies and arrangements are possible.

The communication link 170 may provide for data transmission between the IPG 112 and the external system 130. The transmitted data may include, for example, real-time sensor signals acquired by and stored in the IPG 112, therapy history data, data indicating device operational status of the IPG 112, one or more programming instructions to the IPG 112 which may include configurations for sensing physiological signal or stimulation commands and stimulation parameters, or device self-diagnostic test, among others. In some examples, the IPG 112 may be coupled to the external system 130 further via an intermediate control device, such as a handheld external remote control device to remotely instruct the IPG 112 to generate electrical stimulation pulses in accordance with selected stimulation parameters produced by the external system 130.

The wearable electronic device 120 can include a smart watch, a smart wristband, or a smart ring, among other smart wearables. The wearable electronic device 120 can include a processor to execute a software application (a wearable "app") to collect physiological and functional information from the patient, and process such information to generate one or more physiological or functional parameters. The wearable electronic device 120 can include, or be associated with, one or more sensors to sense physiological or functional information from the patient. Examples of the physiological information may include cardiac electrical activity, electrocardiograms (ECGs), heart rate, heart rate variability (HRV), a photoplethysmography (PPG) signal, blood oxygen saturation (SpO2) such as derived from the PPG signal, blood pressure, a respiration signal, respiratory rate, tidal volume, galvanic skin response, maximum rate of oxygen consumption (VO2$_{max}$) during exercise, peripheral body temperatures, among others. Examples of the functional information may include motor activities such as posture, gait, balance, or physical activities, among others. The functional information may also include sleep or awakening state. Chronic pain patients may experience frequent disrupted sleep or change of sleep patterns. The functional information may be sensed using one or more motions sensors, such as an accelerometer (e.g., a three-axis accelerometer to measure and analyze separate orthogonal components of complex accelerations), a gyroscope (which may be a one-, two-, or three-axis gyroscope), a magnetometer (e.g., a compass), an inclinometer, a goniometer, an electromagnetic tracking system (ETS), or a global positioning system (GPS) sensor, among others. In some examples, the motion sensors may detect frequency or duration of sleep position switch, sleep incline, or other indicators of sleep quality. The wearable app on the wearable electronic device 120 can display the collected or processed physiological and functional information on a user interface, and receive patient input from a user interface.

The wearable electronic device 120 can be communicatively coupled to the mobile device 140 via the a short-range communication link 194, such as Wi-Fi, ZigBee, or Bluetooth, among other wireless communication modes. The physiological and functional information received and processed by the wearable electronic device 120 can be transmitted to the mobile device 140. The mobile device 140 can be a personal mobile electronic device operable by the patient, such as a smart phone, a tablet, or a laptop computer, among other mobile computing devices. The mobile device 140 can execute a software application (a mobile "app") acting as a hub for patient data collection from various sources. For example, physiological and functional information collected and processed by the wearable electronic device 120 can be provided to the mobile device 140 via the short-range communication link 194. Data collected and processed by the IPG 112, including the physiological or functional data, device usage and diagnostic data, and therapy data (e.g., neuromodulation therapy such as SCS for pain relief), can be provided to the mobile device 140 via a short-range communication link 192, such as Wi-Fi, ZigBee, or Bluetooth, among other wireless communication modes.

The mobile app on the mobile device 140 can receive patient reported outcome (PRO). The PRO can be provided in various formats including, for example, textual, graphical, audio, video, among other or a mixture of formats. The PRO can include patient response to a questionnaire. Various questionnaires may be provided to the patient, such as displayed on a user interface of the mobile device 140. The patient response to such questionnaires may be valuable to the clinicians managing the patient. In an example, the mobile device 140 can present to the patient a questionnaire on pain sensation. The mobile app can receive patient description or pain or paresthesia sensation (e.g., intensity, severity, duration, body location, or pattern of pain or paresthesia). In an example, the PRO of pain sensation may include the patient's quantitative rating of pain level on a numerical scale (e.g., 0-10). In some examples, the PRO may include a pain drawing including the patient's depiction or identification of pain locations on the body and optionally pain quality description, or a paresthesia drawing including patient depiction or identification of body locations where paresthesia from stimulation is felt, and optionally paresthesia quality description. In some examples, the PRO may include a questionnaire about patient physical and functional capacities and mobility status, such as intensities, durations, qualities, types or contexts of physical activities the patient engages daily, sleep qualities, mood or emotional or mental health status, or general health status and quality of life reports, among others. In some examples, the PRO may include a patient report on their compliance with a daily routine or a clinical protocol.

In some examples, a questionnaire may additionally or alternatively be presented on the wearable electronic device 120, such as a smart watch. The wearable electronic device 120 can execute a wearable app to receive PRO, such as patient response to the questionnaire, via the user interface of the smart watch. For example, the patient may use the smart watch bezel to select and control a user-interface control element, and respond to the questionnaire. The patient may use the smart watch bezel to rotate a pain drawing at different viewing perspectives and displaying different body locations. In another example, the patient may use the smart watch bezel to select colors, or adjust one or more color properties (e.g., saturation, tone, brightness, or hue) to distinguish different pain levels or at different body locations on a pain drawing.

In some examples, physiological and functional information may be collected from the patient using one or more stationary sensors 125 in an environment of patient's daily life such as at a bedside, in a room at patient home, or in a testing room at a clinic or medical facility. In an example, a motion sensor may be mounted on a chair, a bed (e.g., under or attached to a mattress), or a fixture in a patient's environment. Unlike the wearable sensors which are ambulatory in nature, the stationary sensors 125 can detect one or more functional signals when the patient enters, or remains within, an environment within the scope of surveillance of the stationary sensors 125. Examples of the stationary sensors 125 may include a camera or a video recorder configured to capture an image, an image sequence, or a video of the patient at a specific physical state, such as sitting, standing, walking, or doing physical activities. In an example, the camera may be an infrared camera. In an example, the camera is a digital camera that may generate digital data representation of an image or a video sequence. Functional parameters such as patient posture, gait, balance, and range of motion can be generated from the image data image.

In an example, the stationary sensors 125 can include a weight sensor in a smart scale that can measure body weight of the patient. Chronic pain and obesity are common comorbidities. Reducing body weight may help reduce chronic pain levels. Monitoring patient body weight can be beneficial for managing chronic pain in a patient. In some examples, the stationary sensors 125 can measure or estimate body compositions including, for example, body fat percentage, body mass index (BMI), among others. Body fat percentage can be correlated to pain levels. A higher body fat levels could lead to deconditioning of pain pathways. Reliable body fat measurement at home can provide value to a monitoring physician, particularly if they are advising their patient to make necessary lifestyle changes to reduce weight. In an example, a body fat sensor can be an be incorporated into a smart scale. The smart scale includes an excitation signal generator that can sense electrical pulses towards the body. The body fat sensor can measure a responsive or reflected signal, and estimate a body fat percentage based at least on a relative change in electrical pulse strength.

The stationary sensors 125 can include or be associated with circuitry configured to communicate with the mobile device 140 via a short-range communication link 195, such as Wi-Fi, ZigBee, or Bluetooth, among other wireless communication modes. Measurements collected by the stationary sensors 125 (e.g., body weight or body fat percentage) can be transmitted to the mobile device 140 via the short-range communication link 195, and further be routed to the cloud-computing device 150 via the communication link 180.

In an example, the wearable electronic device 120 can access on-demand cloud-based services from a cloud-computing device 150, and process the collected physiological and functional information using the cloud-based services. Similarly, the mobile device 140 may include a communication module, such as a transceiver circuit, to establish data communication with the cloud-computing device 150. The mobile device 140 can securely route the data received from different sources (e.g., the IPG 112, the wearable electronic device 120, or the mobile device 140) to the cloud-computing device via a communication link 180. The mobile app on the mobile device 140, or the wearable app on the wearable electronic device 120, can interpret the PRO, and extract information such as patient subjective pain experience or feedback on pain therapy (e.g., pain characteristics extracted from the pain drawing). In some examples, the mobile app on the mobile device 140, the wearable app on the wearable electronic device 120, or the software system of the external system 130 can process the sensor information (e.g., physiological or functional signals acquired from the patient or from the stationary sensors 125) and optionally the PRO, and generate a mobility metric representing respective times spent in different activity intensity levels associated with a number of different activity types or contexts during a time period (e.g., a day). The mobile apps or the software system of said devices can trend the mobility metric over time, and determine a progress toward a personalized mobility goal of the patient. The trended mobility metric or the progress can be presented on a user interface of any of said devices, or be communicated to the cloud-computing device 150. In some examples, the sensor information and the PRO can be obtained responsive to a neuromodulation therapy delivered to the patient such as for managing chronic pain. The trended mobility metric or the progress toward the mobility goal may be indicative of an efficacy of the neuromodulation therapy. A user (e.g., a clinician or the patient) can adjust the neuromodulation therapy based on the trended mobility metric or the progress toward the mobility goal.

The cloud-computing device 150, also referred to simply as a "cloud", can include a cloud server or a plurality of networked devices. The cloud-computing device 150 can include a cloud storge that can store data transmitted from networked devices such as the mobile device 140, and optionally the external system 130. The cloud-computing device 150 may include a suite of services (e.g., software as a service, platform as a service, infrastructure as a service) allowing for secured, on-demand access by multiple clients (or "tenants"), such as the wearable electronic device 120, the external system 130, the mobile device 140, or one or more user terminals 160 that can be operated by authorized users from different locations, such as clinicians, medical professionals, or device manufacturer representatives.

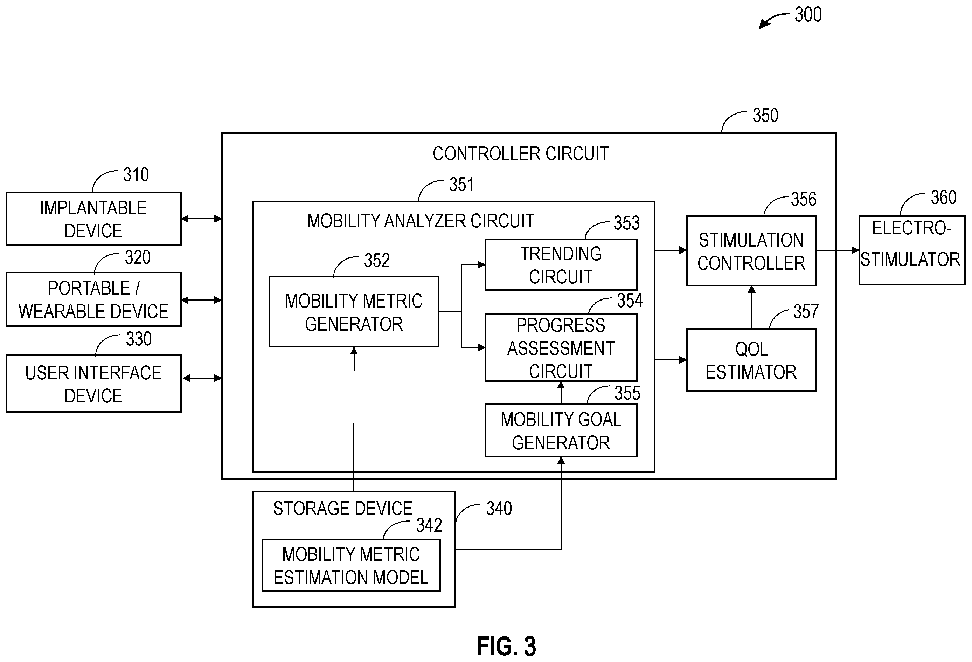

The cloud-based services may include data analysis services for analyzing data received from various sources (e.g., devices or user input), establishing a correspondence between one or more physiological or functional states and one or more pain levels, detecting a physiological or functional state of the patient, detecting or predicting a pain level for the detected patient physiological or functional state using the established correspondence, storing data in the cloud storage, making recommendations to the patient (e.g., making behavior changes to promote pain relief), or making therapy recommendations to the health care provider (e.g., adjusting neuromodulation device setting), performing regular or scheduled device integrity testing or trouble-shooting, making or recommending adjustment to device operations with regard to data acquisition, or formatting and presenting data such as on a display. For example, the patient may use the mobile app on the mobile device 140 to securely access the cloud storage to review the stored data, or to access one or more cloud-based services to aggregate and process patient physiological or functional data and review the data. An authorized user (e.g., a clinician) may use an application installed on one of the user terminals 160 to securely access one or more cloud-based services to review the data, and to modify a device parameter for the IPG 112 such as to adjust pain therapy. Examples of the cloud-based services are discussed below such as with reference to FIGS. 3-8.

Portions of the patient monitoring and pain management system 100 may be implemented using hardware, software, firmware, or combinations thereof. For example, portions of the IPG 112 or portions of the cloud-computing device 150 may be implemented using an application-specific circuit that may be constructed or configured to perform one or more particular functions, or may be implemented using a general-purpose circuit that may be programmed or otherwise configured to perform one or more particular functions. Such a general-purpose circuit may include a microprocessor or a portion thereof, a microcontroller or a portion thereof, or a programmable logic circuit, or a portion thereof. For example, a "comparator" may include, among other things, an electronic circuit comparator that may be constructed to perform the specific function of a comparison between two signals or the comparator may be implemented as a portion of a general-purpose circuit that may be driven by a code instructing a portion of the general-purpose circuit to perform a comparison between the two signals.

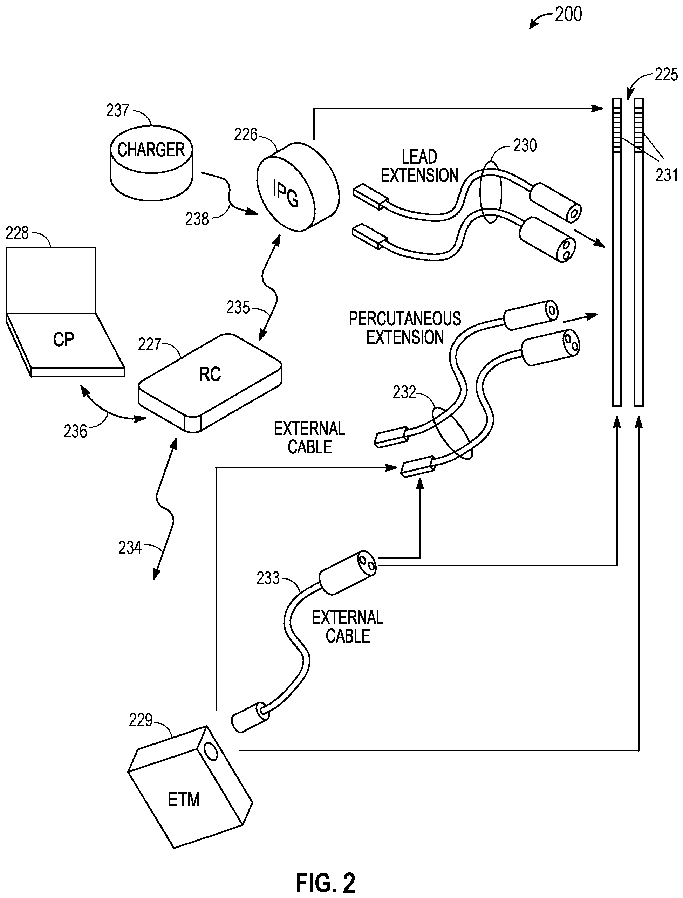

FIG. 2 illustrates, by way of example, an example of a Spinal Cord Stimulation (SCS) system 200, also referred to as a Spinal Cord Neuromodulation system, which is an example of the implantable system 110. The SCS system 200 can include a plurality (illustrated as two) of implantable neuromodulation leads 225, an implantable pulse generator (IPG) 226, an external RC 227, a clinician's programmer (CP) 228, and an external trial modulator (ETM) 229. The IPG 226 may be physically connected via one or more percutaneous lead extensions 230 to the neuromodulation leads 225, which carry a plurality of electrodes 231. As illustrated, the neuromodulation leads 225 may be percutaneous leads with the electrodes arranged in-line along the neuromodulation leads. Any suitable number of neuromodulation leads can be provided, including only one, as long as the number of electrodes is greater than two (including the IPG case function as a case electrode) to allow for lateral steering of the current. Alternatively, a surgical paddle lead can be used in place of one or more of the percutaneous leads. The IPG 226 includes pulse generation circuitry, also referred to as a pulse generator, that delivers electrical neuromodulation energy in the form of a pulsed electrical waveform (*i.e.*, a temporal series of electrical pulses) to the electrodes in accordance with a set of neuromodulation parameters.

The ETM 229 may also be physically connected via the percutaneous lead extensions 232 and external cable 233 to the neuromodulation leads 225. The ETM 229 may have similar pulse generation circuitry as the IPG 226 to deliver electrical neuromodulation energy to the electrodes in accordance with a set of neuromodulation parameters. The ETM 229 is a non-implantable device that is used on a trial basis after the neuromodulation leads 225 have been implanted and prior to implantation of the IPG 226, to test the responsiveness of the neuromodulation that is to be provided. Functions described herein with respect to the IPG 226 can likewise be performed with respect to the ETM 229.

The RC 227 may be used to telemetrically control the ETM 229 via a bi-directional RF communications link 234. The RC 227 may be used to telemetrically control the IPG 226 via a bi-directional RF communications link 235. Such control allows the IPG 226 to be turned on or off and to be programmed with different neuromodulation parameter sets. The IPG 226 may also be operated to modify the programmed neuromodulation parameters to actively control the characteristics of the electrical neuromodulation energy output by the IPG 226. A clinician may use the CP 228 to program neuromodulation parameters into the IPG 226 and ETM 229 in the operating room and in follow-up sessions.

The CP 228 may indirectly communicate with the IPG 226 or ETM 229, through the RC 227, via an IR communications link 236 or another link. The CP 228 may directly communicate with the IPG 226 or ETM 229 via an RF communications link or other link (not shown). The clinician detailed neuromodulation parameters provided by the CP 228 may also be used to program the RC 227, so that the neuromodulation parameters can be subsequently modified by operation of the RC 227 in a stand-alone mode (i.e., without the assistance of the CP 228). Various devices may function as the CP 228. Such devices may include portable devices such as a lap-top personal computer, mini-computer, personal digital assistant (PDA), tablets, phones, or a remote control (RC) with expanded functionality. Thus, the programming methodologies can be performed by executing software instructions contained within the CP 228. Alternatively, such programming methodologies can be performed using firmware or hardware. In any event, the CP 228 may actively control the characteristics of the electrical neuromodulation generated by the IPG 226 to allow the desired parameters to be determined based on patient feedback or other feedback and for subsequently programming the IPG 226 with the desired neuromodulation parameters. To allow the user to perform these functions, the CP 228 may include a user input device (e.g., a mouse and a keyboard), and a programming display screen housed in a case. In addition to, or in lieu of, the mouse, other directional programming devices may be used, such as a trackball, touchpad, joystick, touch screens or directional keys included as part of the keys associated with the keyboard. An external device (e.g. CP) may be programmed to provide display screen(s) that allow the clinician to, among other functions, to select or enter patient profile information (e.g., name, birth date, patient identification, physician, diagnosis, and address), enter procedure information (e.g., programming/follow-up, implant trial system, implant IPG, implant IPG and lead(s), replace IPG, replace IPG and leads, replace or revise leads, explant, etc.), generate a pain map of the patient, define the configuration and orientation of the leads, initiate and control the electrical neuromodulation energy output by the neuromodulation leads, and select and program the IPG with neuromodulation parameters in a surgical or clinical setting.

An external charger 237 may be a portable device used to transcutaneously charge the IPG via a wireless link such as an inductive link 238. Once the IPG has been programmed, and its power source has been charged by the external charger or otherwise replenished, the IPG may function as programmed without the RC or CP being present.

FIG. 3 illustrates generally an example of a mobility monitor system 300, which is an example of the patient monitoring and pain management system 100. The system 300 may be used to monitor and analyze patient mobility, among other physiological and functional parameters, such as in the presence of pain or pain treatment (e.g., neuromodulation therapy). The system 300 may make recommendation of behavior changes to promote pain relief, or to determine whether to recommend therapy adjustment or initiate automatic therapy adjustment based on the patient mobility.

The system 300 may include a controller circuit 350 and one or more devices communicatively coupled thereto, including, for example, an implantable device 310, a portable or wearable device 320, or a user interface device 330. The system 300 may also include a storage device 340 and an electrostimulator 360. The implantable device 310 can include, for example, the IPG 112. The portable or wearable device 320 can include, for example, the mobile device 140 and/or the wearable electronic device 120 (e.g., a smart watch). The user interface device 330 can include, for example, one or more of the clinician's programmer (CP) 228, the mobile device 140, the wearable electronic device 120, or the user terminal 160. Although the controller circuit 350 is shown to be separated from the implantable device 310, the portable or wearable device 320, and the user interface device 330, this is by way of example and not limitation. In some examples, the controller circuit 350 or a portion thereof can be implemented in at one of the implantable device 310, the portable or wearable device 320, or the user interface device 330.

The controller circuit 350 may be implemented as parts of a microprocessor circuit, which may be a dedicated processor such as a digital signal processor, application specific integrated circuit (ASIC), microprocessor, or other type of processor for processing information including physical activity information. Alternatively, the microprocessor circuit may be a general-purpose processor that may receive and execute a set of instructions of performing the functions, methods, or techniques described herein.

The controller circuit 350 may include circuit sets comprising one or more other circuits or sub-circuits, such as one or more of a mobility analyzer circuit 351, a stimulator controller 356, or a quality of life (QoL) estimator 357. The circuits or sub-circuits may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

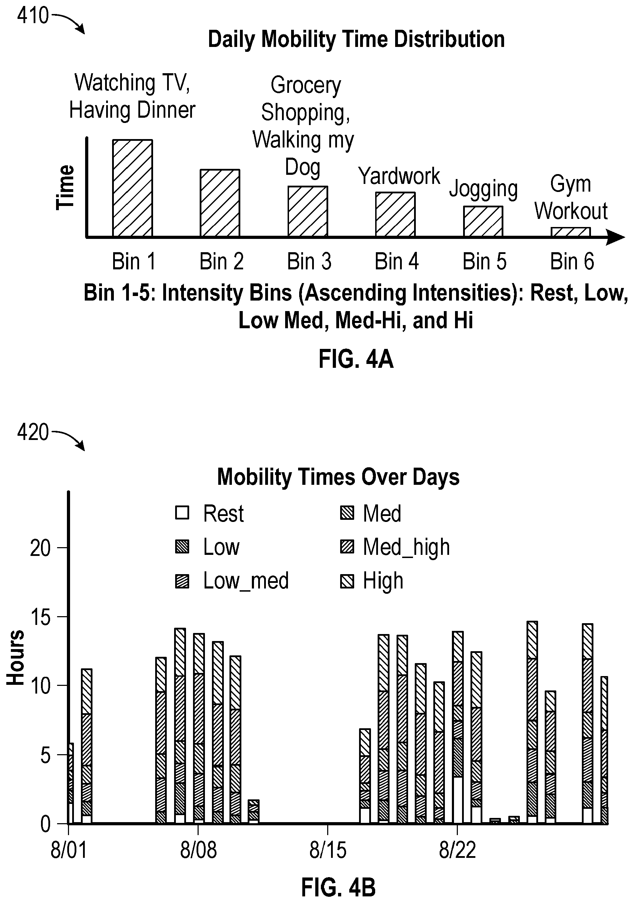

The mobility analyzer circuit 351 can receive physiological or functional signals, optionally along with the patient reported outcome (PRO) as discussed above with reference to FIG. 1, from one or more of the implantable device 310, the portable or wearable device 320, or the user interface device 330. The mobility analyzer circuit 351 can include a mobility metric generator 352 that can generate a mobility metric from the received physiological or functional signals and optionally the PRO. In an example, the received physiological or functional signals may include physical activity signals sensed by an activity sensor, such as an accelerometer (XL) included in or otherwise communicatively coupled to the implantable device 310 (e.g., the IPG 112) or the portable or wearable device 320 (e.g., the mobile device 140 or the wearable electronic device 120). The mobility metric may be represented by respective mobility times spent in different activity intensity levels associated with one or more activity types or contexts during a time period (e.g., a day, a number of days, a week, or other user-specified time period), hereinafter referred to as the "mobility time distribution." In an example, the activities intensities may be categorized into one or a plurality of mobility intensity bins, such as based on a comparison of the XL signal intensity (e.g., amplitude or signal power) to one or more thresholds or threshold ranges. By way of example, FIGS. 4A-4C illustrate examples of mobility metric represented by mobility time distribution and mobility scores. Specifically, FIG. 4A illustrates a diagram 410 of a distribution (represented by a histogram) of mobility times (on the y-axis) across six mobility intensity bins (on the x-axis) corresponding to rest (bin 1), low intensity (bin 2), low-medium intensity (bin 3), medium intensity (bin 4), medium-high intensity (bin 5), an high intensity (bin 6). The distribution of mobility time as shown in FIG. 4A may be generated using activity data collected over a 24-hour period, and is referred to a daily mobility distribution. The distribution of mobility times can be displayed on a user interface, and presented in a format of a bar chart, a pie chart, or textual formats. FIG. 4B illustrates a diagram 420 of daily mobility distribution (y-axis) over a number of days (x-axis) in a format of a stacked bar chart, where each daily mobility distribution is represented as a number of sub-bars vertically stacked end to end. Each sub-bar represents a mobility time at a mobility intensity bin, such as one of bin 1 through bin 6 shown in FIG. 4A. The height of the sub-bar is proportional to the mobility time at that intensity bin. In an example, the sub-bars can be color-coded or shown in different patterns to visually distinguish different intensity bins.

The mobility metric generator 352 can assign a numerical intensity value for each of the intensity bins such that. In an example, a higher intensity value can be assigned to a higher intensity bin. The metric generator 352 can compute a mobility score (S(I)) using a weighted sum of the intensity values (I) each scaled by respective weight factors (w), across all (e.g., N) the intensity bins:

$$S(I) = \sum_{i=1}^{N} w_i I_i \qquad (1)$$

The weight factors in Equation (1) above can be determined based on the respective mobility times (T) at corresponding intensity bins. In an example, the weight factors can be proportional to the mobility times, such that a longer mobility time corresponds to a larger weight factor, while a shorter mobility time corresponds to a smaller weight factor.

Alternatively, the mobility score (S(T)) may be computed using a weighted sum of the mobility times (T) each scaled by respective weight factors (w), across all (e.g., N) the intensity bins:

$$S(T) = \sum_{i=1}^{N} w_i T_i \qquad (2)$$

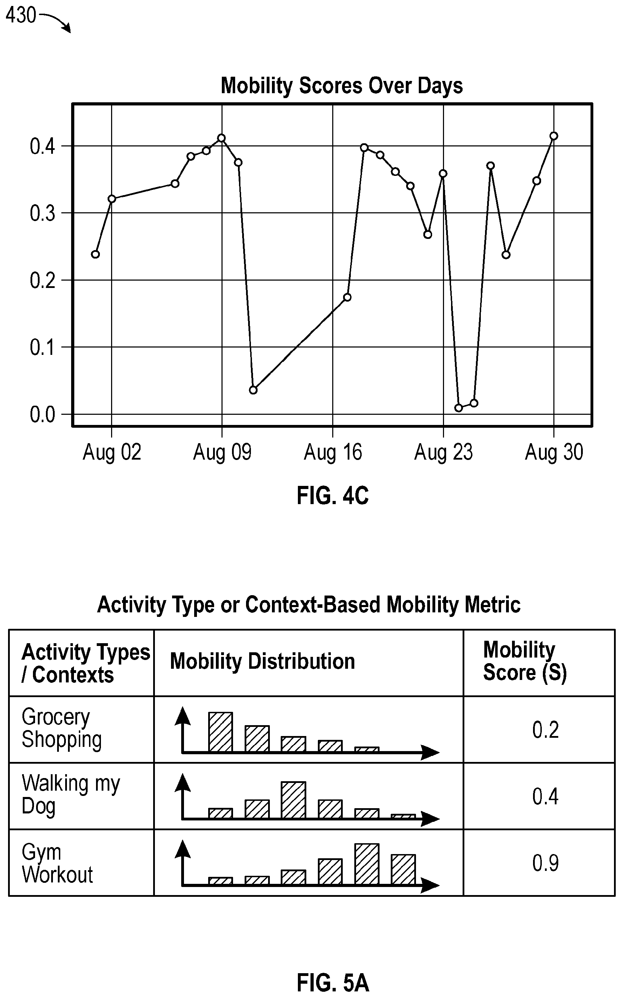

The weight factors in Equation (2) above can be determined based on the respective mobility intensity values (I) at corresponding intensity bins. In an example, the weight factors can be proportional to the mobility intensity values, such that a higher intensity value corresponds to a larger weight factor, while a lower intensity value corresponds to a smaller weight factor. The mobility score (S) calculated according to Equation (1) or (2) indicates an overall mobility during the evaluation time period (e.g., a day). In an example, the mobility score (S) can be normalized or otherwise converted to a value between 0 and 1, where 1 indicates the highest possible mobility capacity and 0 indicates the lowest possible mobility capacity. FIG. 4C illustrates a diagram 430 of daily mobility scores (y-axis) over a number of days (x-axis).

In some examples, the metric generator 352 can compute a dispersion metric of the mobility times (T) across the plurality of (e.g., N) intensity bins. An example of such dispersion metric is an entropy (H(T)) of the mobility times (T):

$$H(T) = -\sum_{i=1}^{N} P(T_i)\, log P(T_i)$$

$$(3)$$

where $P(T_i)$ represents the probability of the $T_i$ (corresponding to the i-th intensity bin), and can be estimated as a

proportion of $T_i$ over the entire observation period, that is, $P(T_i) \approx T_i / \sum_{i=1}^{N} T_i$. The mobility time entropy (H(T)) indicates the randomness of distribution of mobility times across different intensity bins. For example, a higher entropy may result from mobility times that spread over all intensity bins than if the mobility times are skewed to one or more intensity bins. As will be discussed below, in some examples, a personalized mobile goal (such as generated by the mobility goal generator 355) may be characterized by mobility times more evenly distributed across all intensity bins, which corresponds to a high entropy goal (H*(T)). If the patient's daily mobility time entropy is less than the entropy goal H*(T), the metric generator 352 may determines that the mobility times are skewed towards certain intensity bins but not others. The patient may be recommended (e.g., via the portable or wearable devices 320 or the user interface device 330) to spread the mobility times across a wider range of mobility intensity bins.

Although the accelerometer-based activity signals can be used to provide a "standard" mobility metric of the patient, in some occasions or in some patients, such accelerometer-based mobility metric may be unavailable to use (e.g., devices lack a 3-axis accelerometer, or a device having the accelerometer is not worn or hold by the patient), or unreliable to use (e.g., due to interferences or noises). In such cases, it would be desirable to use other on-board physiological or functional sensors to derive a "surrogate" mobility metric comparable to the standard, accelerometer-based mobility metric. According to some embodiments, the metric generator 352 can estimate a mobility metric by applying a physiological or functional signal, acquired by a physiological or functional sensor other than an accelerometer, to at least one trained mobility metric estimation model 342. Examples of such physiological or functional signals may include electrocardiograms (ECGs), heart rate, heart rate variability, a photoplethysmography (PPG) signal, a blood oxygen saturation (SpO$_2$) signal, a blood pressure signal, a respiration signal, respiratory rate, tidal volume, galvanic skin response, maximum rate of oxygen consumption (VO2$_{max}$) during exercise, peripheral body temperatures, a posture, or a sleep signal, electrograms (e.g., cardiac electrophysiological signal sensed within the heart), heart sounds, among others. The estimation model 342 may be trained and calibrated against the accelerometer-based mobility metric values, such that the "surrogate" mobility metric estimated from various physiological or functional signal may be comparable to the standard, accelerometer-based mobility metric within a specified tolerance level (e.g., $\pm$ 5% or $\pm$ 10%). The mobility metric estimation model 342 may be trained using a training dataset comprising a set of activity signals sensed by accelerometers and a set of physiological signals sensed by physiological sensors different from the accelerometers. The set of activity signals and the set of physiological signals are sensed substantially concurrently from the patient (e.g., within an hour or within the same day). The training dataset may further comprise accelerometer-based mobility metric values that are determined from the set of activity signals and used as "labels" during training. Various training algorithms may be used train the mobility metric estimation model 342, such as Logistic Regression (LR), Naive-Bayes, Random Forest (RF), neural networks (NN), deep neural networks (DNN), matrix factorization, or Support Vector Machines (SVM), among other supervised learning or unsupervised learning. In an example, the mobility metric estimation model 342 can be trained using deep learning. The ML model has an architecture of a deep neural network Examples of DNN include a convolutional neural network (CNN), a recurrent neural network (RNN), a deep belief network (DBN), a long-term and short-term memory (LSTM) network, a transfer learning network, or a hybrid neural network comprising two or more neural network models of different types or different model configurations. The trained models can be stored in a storage device 340. As the variety of physiological sensors may be included in different devices (e.g., the IPG 112, the mobile device 140, or the wearable electronic device 120), using the trained mobility metric estimation model enables convenient, efficient, and high-fidelity mobility assessment through a common mobility estimation platform that enables a "plug-and-play" capability from a range of existing devices or sensors, yet without requiring dedicated, device- or sensor-specific mobility algorithms and software. This can improve mobility assessment efficiency the reduce overall development and operation cost.

The metric generator 352 may apply physiological or functional signals to the respective trained mobility metric estimation model 342 to generate, for example, heart rate-based mobility metric, SpO$_2$-based mobility metric, respiration rate-based mobility metric, etc. In some examples, the metric generator 352 can identify subsets (e.g., minimal subsets) of physiological or functional signals that are sufficient to enable accurate estimation of the mobility metric, such that the "surrogate" mobility metric is comparable to the standard, accelerometer-based mobility metric within the specified tolerance level. Any future devices that can provide at least the physiological or functional signals from one of these minimal subsets (within predetermined accuracy ranges) can be used to calculate the "surrogate" mobility metric without new algorithm development effort.

In some examples, the mobility metric generator 352 can receive information about activities that the patient engages in during which the activity signal or other physiological or functional signals are collected. Examples of such activity information include activity intensity, activity timing information (e.g., start time and end time), activity type or context (e.g., brisk walk, gym workout, grocery shopping, doing yardwork, walking the dog, cooking a meal, washing dishes, morning jogging, hiking, etc.), among other information. In an example, such activity information may be received as user input via, for example, the portable or wearable device 320 or the user interface device 330. For example, when the patient is about to start a particular type of activity (e.g., gym workout), he/she may provide an input regarding the type or context of the activity, activity start time and end time, etc. The user input can be in various forms, such as textual or voice input. A natural language processing (NLP) system (which can be included in the portable or wearable device 320, the user interface device 330, or the controller circuit 350) can process the textual or voice input to determine an association or a correlation between the description of the activity (e.g., words and or their frequencies) and the mobility metric (e.g., time spent at different activity intensities, or mobility scores). The physiological or functional signals acquired during the activity and the mobility metric generated therefrom can be "tagged" with the activity type or context provided by the patient.

In addition or alternative to user provided activity information, in some examples, a device (e.g., the portable or wearable device 320, such as the mobile device 140, or the wearable electronic device 120) can automatically determine or infer the activity information based on a predetermined patient daily schedule. For example, in accordance with a patient's schedule of daily gym workout from 8:00 a.m. to 9:00 a.m., the device may automatically activate data collection (of physiological or functional signals) at the scheduled start time, and associate the collected data with the activity type or context per patient schedule. This feature is also referred to "mobility tracking." The mobility tracking may be modified (e.g., allow certain activities to be tracked but not other activities) or turned off by the patient. The patient may provide a reason for the modification, such as being sick. In some examples, the device can automatically determine or infer the activity information (e.g., type or context of activities) based on patient locations (e.g., gym, park, grocery store) automatically (with user consent) detected by a GPS sensor in a mobile device or smart wearable device, or obtained from other location services. For example, when the device detects the patient is in a gym, the device may automatically activate data collection (of physiological or functional signals), and associate the collected data and the mobility metric generated therefrom with the activity type or context inferred from patient locations.

The mobility metric generator 352 can generate a patient-specific association model that establishes an association between the activity information (e.g., activity type or context either provided manually by the user or automatically determined or inferred by a device) and the mobility metric (e.g., mobility times spent at different activity intensity bins derived the physiological or functional signals acquired during the activity, or mobility scores). The association may be presented to the patient to signify different activity types or contexts and their relationships to the overall mobility status, and to encourage positive change in daily behavior (e.g., increase or decrease the amount of time spent in certain activities so as to have a more balanced mobility distribution across a broad spectrum of intensities, which may help alleviate pain and improve overall quality of life). Additionally or alternatively, the association may be presented to other patients to educate them on how to achieve desired mobility score or to reach a personalized mobility goal, aimed at improving their general health and/or pain outcomes.

In an example, as shown in FIG. 4A, information about activity type or context can be displayed on the mobility distribution plot and associated with appropriate activity intensity bins, such as "Watching TV" or "Having dinner" associated with the lowest activity bin (bin 1), "Grocery shopping" and "Walking my dog" associated with low-medium activity bin (bin 3), "Yardwork" associated with medium activity bin (bin 4), "Jogging" associated with medium-high activity bin (bin 5), and "Gym workout" associated with the high activity bin (bin6). As activity type or context are more intuitive than activity intensity levels or bins, associating activity types or contexts with intensity bins as shown in FIG. 4A provides a direct visual feedback on respective times spent on various different activities. The patient may be given more meaningful recommendations, such as spending more time in certain types of activity (e.g., "Jogging" or "Gym workout" corresponding to intensity bins 5 and 6) on which the patient spent less time in the past.

In some examples, the mobility metric generator 352 may generate respective mobility metrics for each of a number of different activity types or contexts. FIG. 5A illustrates an example of such activity type- or activity context-based mobility metric. Mobility distributions (similar to that shown in FIG. 4A) can be generated respectively for different activity types including, by way of example and not limitation, "Grocery shopping", "Walking my dog", and "Gym workout", over a predetermined time period, such as a week or a month in this example. Such activity type- or context-based mobility metric can improve interpretation of the mobility, and enable more meaningful data comparison (e.g., comparing patient current and past mobility when engaging in a particular activity, or comparing to a mobility goal) to signify a progress or an improvement in patient mobility. For example, if "yardwork" or "walking my dog" (e.g., detected from patient voice or textual input and identified by an NLP system) are found to be associated with a high mobility score (e.g., at or above 75th percentile of the population-based mobility score), the system may generate and display on a user interface of the portable or wearable device 320, or the user interface device 330 a recommendation to the patient to engage in such types of activities more frequently or for a longer duration each time, as a higher mobility score may be associated with pain reduction and improvement in patient quality of life. An example of such recommendation can be: "Have you done any yardwork or walked your dog recently? Your system has identified that when you're active doing yardwork or walking your dog, your pain tends to improve."

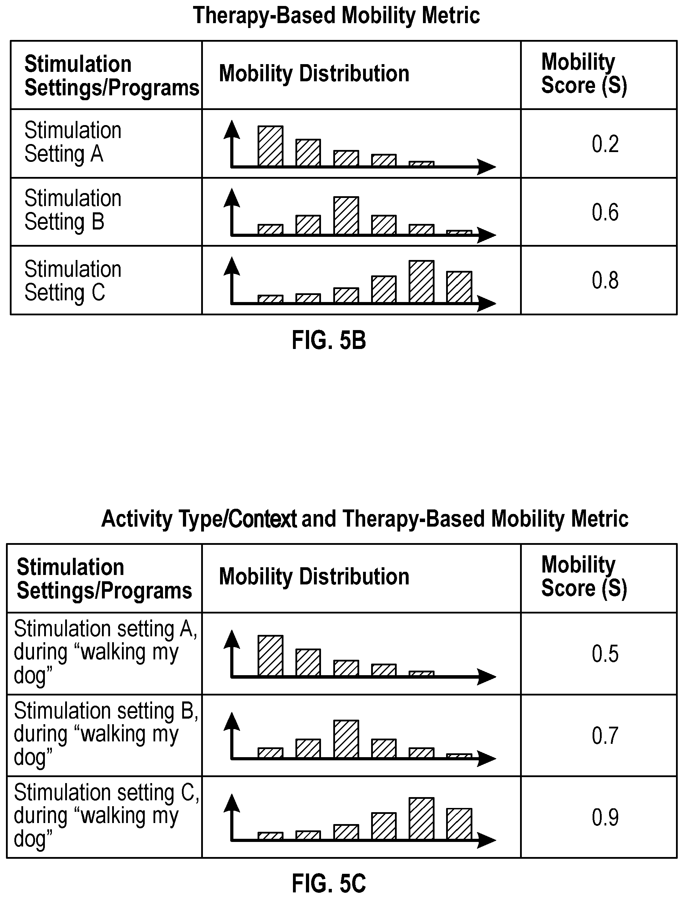

In some examples, physiological or functional signals (and optionally the PRO information) can be collected in response to a neuromodulation therapy, such as SCS for chronic pain control. The neuromodulation therapy may be delivered in accordance with a stimulation setting, or one of a number of predetermined stimulation programs. The stimulation setting can be represented by one or more stimulation parameters taken respective values, including, for example, pulse amplitude, pulse width, pulse frequency, pulse waveform or shape, electrode configurations (electrodes designated as anode or cathode) and stimulation current or energy fractionalization across electrodes, etc. A stimulation program represents a collection of stimulation parameters with predetermined values. Examples of the stimulation programs can include I3D, Contour, FAST, or Micro Burst programs, among other programs. FIG. 5B illustrates an example of mobility metric (e.g., mobility time distribution or mobility score) respectively evaluated for each of a number of different stimulation settings or stimulation programs. Mobility distributions (similar to that shown in FIG. 4A) can be generated respectively for different stimulation settings or programs (e.g., setting A, B, and C as shown) over a predetermined time period, such as a week or a month in this example. Such stimulation setting or program-based mobility metric can improve interpretation of the impact of stimulation settings or programs on patient mobility, and enable more efficient individualized optimization of neuromodulation therapy. For example, based on a comparison of the mobility metrics under different stimulation settings A, B, and C in FIG. 5B, the stimulation controller 356 can select an "optimal" stimulation setting with the corresponding signal metric satisfying a specific condition, such as the one with the largest mobility score or the largest mobility entropy, or the one with corresponding mobility times most spreading out among different mobility intensity bins. The stimulation controller 356 can generate a control signal to the electrostimulator 360 to adjust the neuromodulation therapy using the selected stimulation setting.

In some examples, the stimulation setting or program-based mobility metric as described above with reference to FIG. 5B may be evaluated for a specific activity type or activity context. FIG. 5C illustrates an example of mobility metric respectively evaluated for each of a number of different stimulation settings or stimulation programs (e.g., stimulation settings A, B, and C) when the patient engages the same type of activity or under the same activity context, (e.g., "walking my dog"). The stimulation controller 356 can select a stimulation setting or program for that specific mobility type or context based on a comparison of the mobility metrics. For example, based on a comparison of the stimulation type and setting-based mobility metrics as shown in FIG. 5C, the stimulation controller 356 can select, for future "walking my dog" activity, an "optimal" stimulation setting with the corresponding signal metric satisfying a specific condition, such as the one with the largest mobility score or the largest mobility entropy, or the one with corresponding mobility times most spreading out among different mobility intensity bins. The stimulation controller 356 can generate a control signal to the electrostimulator 360 to adjust the neuromodulation therapy using the selected stimulation setting when the patient engages in the same type of activity (e.g., walking my dog).

In some examples, the mobility metric generator 352 can compare the mobility metric to a population-based mobility metric to determine the patient's relative mobility and function capacity compared to other patients with similar medical conditions or similar demographics to the patient being evaluated (hereinafter referred to as "similar patients"). For example, a chronic pain patient may have a different expectation of mobility, or improvement in mobility capacity responsive to some neuromodulation therapy, than a non-pain patient or a patient with minor, acute pain. In an example, the relative mobility may be represented by a percentile rank in the group of similarly patients. For example, a 75th percentile indicates that the patient being evaluated has a mobility and functional capacity greater than 75% of the group of similar patients. A relative mobility with respect to a patient population allows for an objective assessment of patient mobility status and guided therapy optimization. The population-based mobility metric can be generated using aggregated physiological or functional data collected from a patient group having a common characteristic as the patient, such as a patient group with chronic pain, a patient group with similar diagnosis of underly diseases, a patient group with similar baseline pain, a patient group with similar time of implant of implantable devices (e.g., IPG 112), a patient group with similar demographics (e.g., age) subgroup, or a patient group with similar comorbidities. The aggregated physiological or functional data and the population-based mobility metrics can be stored in the storage device 340. In an example, the mobility metric generator 352 can determine a relative mobility metric represented by a percentile of the population-based mobility metric for a user-specified patient group. For example, a 75-percentile indicates that the patient has a mobility metric (e.g., a mobility score) higher than 75% of patients in that group. In some examples, the mobility score may be scaled by the aggregated, population-based data. For example, the patient raw mobility score may be normalized by the population-based mobility score aggregated over a patient population.

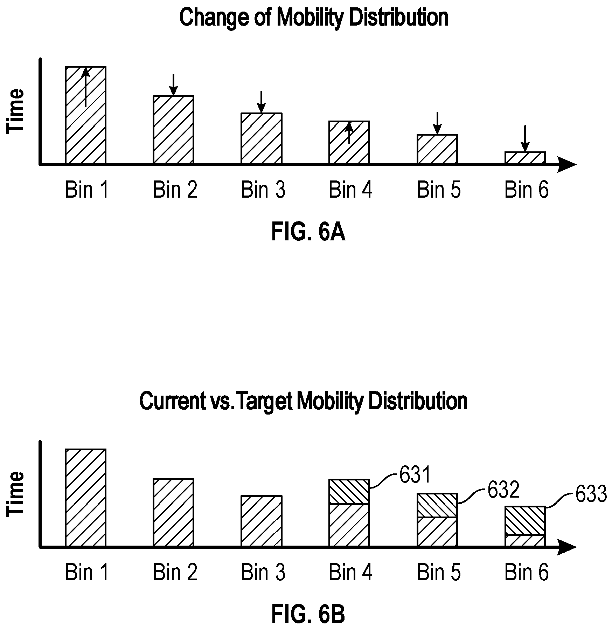

Referring back to FIG. 3, the mobility analyzer circuit 351 can include a trending circuit 353 to trend the mobility metric over time. The mobility trend can be computed as a trend of mobility score (e.g., using Equation (1) or (2)), a trend of entropies (e.g., using Equation (3)), or a trend of mobility time at one or more activity intensity bins over a number of days, weeks, or months. In another example where the mobility metric is represented by a mobility distribution as shown in FIG. 4A, the mobility trend can be represented by a change in mobility distribution over time, including differences in mobility times associated with respective one or more activity intensity bins. By way of example and not limitation, FIG. 6A illustrates a graphical representation of a change from a previous mobility distribution (e.g., a day or a week ago). The directions of change in mobility times in respective activity intensity bins can be graphically shown using arrows on each mobility time bars, such as an upward arrow indicating an increase in mobility time, or a downward arrow indicating decrease in mobility time.

In some examples, the trending circuit 353 can trend the mobility metric since implantation of the implantable device 310, or since the patient received first neuromodulation therapy. The mobility trend can indicate how patient respond to the neurostimulation therapy, such as an improvement in overall mobility capacity (e.g., increase in mobility score over time), or an improvement in certain intensity levels (intensity bins) or certain types of activities over time.

The mobility analyzer circuit 351 can include a progress assessment circuit 354 to determine a progress toward a personalized mobility goal of the patient. The mobility analyzer circuit 351 can include a mobility goal generator 355 to determine the personalized mobility goal based at least in part on a baseline mobility metric of the patient during a baseline time period. The baseline mobility metric can be determined during a controlled baseline mobility test when the patient performs a number of activities of different types and intensities during a specified testing period (e.g., 2 weeks, 4 weeks, or other user-specified duration). The baseline mobility test can provide information on a normal range of mobility metric values for the patient, defined by a maximum metric value and a minimum metric value. In an example, the baseline mobility metric may include daily mobility metric values (e.g., daily mobility score S computed using Equation (1) or (2)) during a number of days, and the personalized mobility goal ($S_g$) may be determined to be the maximum daily mobility metric value ($S_{max}$) scaled by a weight factor (w), that is, $S_g = w*S_{max}$. The weight factor (w) can be a programmable value greater than one (e.g., w = 1.25). In another example, the weight factor can be determined based on the patient's baseline pain score and a goal pain score (or the pain score reduction goal). For example, if the baseline pain score is 8, the goal pain score is 2 (or the reduction goal of 6), then the weight factor can be determined to be w = 1+ (8-2)/8 =1.75.

In addition or alternative to the patient baseline mobility metric, other metrics may be relied upon to determine or adjust the personalized mobility goal. In an example, the mobility goal generator 355 may determine the personalized mobility goal based on a population-based mobility metric ($S_{op}$) generated from a group of patients having similar medical conditions or similar demographics to the patient being evaluated. The population-based mobility metric ($S_{pop}$) may be determined as an average, median, or maximum mobility metric value across the group of such "similar" patients. In an example, the personalized mobility goal ($S_g$) may be determined to be the population-based mobility metric ($S_{pop}$) scaled by a weight factor (w), that is, $S_g = w*S_{pop}$.

The mobility goal generator 355 may determine or adjust the personalized mobility goal based on patient progress towards an existing mobility goal. For example, if the patient hits the existing mobility goal, the mobility goal may be updated (e.g., increased by a specific factor, e.g., 1.25). In another example, the mobility goal generator 355 may use the mobility trend generated by the trending circuit 353 to project a future goal-hitting time (e.g. in 2 weeks, or a month), and set a new goal or update the existing goal as approaching the projected goal-hitting time. If the mobility goal generator 355 determines that the patient is not progressing toward the existing goal as planned, the mobility goal generator 355 may reset the mobility goal (e.g., decrease by a specific factor, e.g., 1.25). The mobility goal thus established is not only tailored for individual patient health status, but also enables assessing patient improvement in mobility capacity relative to the group of "similar" patients.

The personalized mobility goal can be represented by a target mobility score, a target mobility time entropy, or a target mobility distribution. The progress assessment circuit 354 can determine patient progress toward the mobility goal based on, for example, a difference between the mobility score (e.g., calculated using Equation (1) or (2)) of the patient and the target mobility score, a difference between the mobility time entropy (e.g., calculated using Equation (3)) and the target mobility time entropy, or a comparison of the calculated mobility distribution (as illustrated in FIG. 4A) to the target mobility distribution. By way of example and not limitation, FIG. 6B illustrates an graphical comparison of a present mobility distribution and a target mobility distribution (which is a part of a personalized mobility goal) across a plurality of activity intensity bins. In this example, the present mobility distribution and the target mobility distribution have comparable mobility times in lower activity intensity bins (e.g., bin 1 through bin 3). In higher intensity bins (e.g., bin 4 through bin 6), the calculated mobility distribution has lower mobility times than the target mobility distribution, shown as shaded sub-bars 631, 632, and 633 on top of mobility time bars in bin 4, bin 5, and bin 6 of the calculated mobility distribution, respectively. As the personalized mobility goal can be determined based at least in part on population-based mobility data, FIG. 6B may be modified to show a comparison of the calculated mobility distribution to a population-based mobility distribution generated using data collected from a group of patients having similar medical conditions or similar demographics to the patient.

The mobility trend (such as that shown in FIG. 6A) and the patient progress toward a personalized mobility goal (such as that shown in FIG. 6B) can be displayed on the user interface device 330 or of the portable or wearable device 320. The mobility trend and the progress toward the personalized mobility goal may be indicative of an efficacy of the neuromodulation therapy.

The stimulation controller 356 can adjust stimulation settings based on the trended mobility metric provided by the trending circuit 353 or the progress toward the mobility goal provided by the progress assessment circuit 354. The stimulation controller 356 can generate the control signal to the electrostimulator 360 to deliver electrostimulation therapy in accordance with the adjusted stimulation setting. In an example, the neuromodulation therapy may include spinal cord stimulation (SCS) for controlling chronic pain. The stimulation controller 356 can adjust stimulation settings in accordance with the trended mobility metric or the progress toward the mobility goal. Examples of the stimulation settings may include, electrode selection and configuration, stimulation parameter values including, for example, amplitudes, pulse width, frequency, pulse waveform, active or passive recharge mode for FAST, ON time, OFF time, and therapy duration for BST, among others. In an example, the stimulation controller 356 can be implemented as a proportional integral (PI) controller, a proportional-integral-derivative (PID) controller, or other suitable controller that takes the trended mobility metric or the progress toward the mobility goal as a feedback on the adjustment of stimulation settings. As the selected signal feature is optimized to distinguish between different therapy states such as an optimal therapy state and an avoidance state, the closed-loop feedback control based on the optimal feature can achieve more reliable paresthesia-free effects and improved patient outcome comprising the therapeutic effects of sub-perception electrostimulation.

The electrostimulator 360 can be an implantable module, such as incorporated within the IPG 112. Alternatively, the electrostimulator 360 can be an external stimulation device, such as incorporated with the ETM 229. In some examples, the user can be notified of therapy reminder or prompted to adjust a stimulation setting, via the RC 227, the portable or wearable device 320, or the user interface device 330. The user can titrate the neuromodulation therapy, via the stimulation controller 356, in accordance with the trended mobility metric or the progress toward the mobility goal. Alternatively, electrostimulation therapy may be automatically delivered in accordance with the adjusted stimulation setting.

The quality of life (QoL) estimator 357 can generate a QoL score based at least in part on the mobility metric. Other information, such as patient reported outcome (PRO), may also be used to calculate the QoL score. The QoL score may be trended over time, and presented to the user along with the trended mobility metric (e.g., a mobility score trend) and optionally along with neurostimulation therapy information (e.g., SCS program usage information). In an example, the QoL estimator 357 may determine a correlation between a sensor-based mobility metric (such as based on the accelerometer or other physiological sensors for sensing heart rate, blood oxygen saturation ($SpO_2$), respiration, posture, or sleep state) and patient-reported functional improvements. The patient-reported functional improvement may include activities of daily living (ADLs), pain interference, Patient-Reported Outcomes Measurement Information System (PROMIS) profile that assesses pain intensity using a single 0-10 numeric rating item and seven health domains (physical function, fatigue, pain interference, depressive symptoms, anxiety, ability to participate in social roles and activities, and sleep disturbance) using four items per domain, be quantified as functional improvement scores. The patient-reported functional improvement may be quantified by numerical physical function scores.

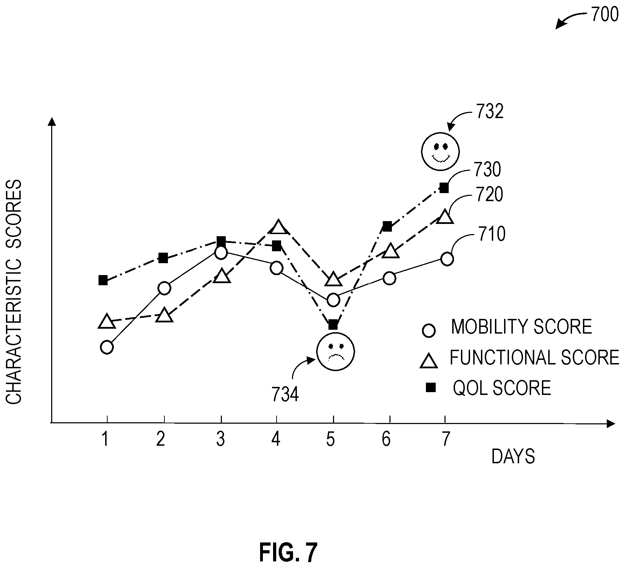

By way of example, FIG. 7 illustrates a diagram 700 of a mobility score trend 710, a physical function score trend 720, and a QoL score trend 730 of the same patient during a specific time period (e.g., 7 days). A correlation between the mobility scores and the physical function scores can be calculated using said trends, and used to predict a future functional state of the patient. The correlation and the QoL score may enable more comprehensive and objective assessment of overall physical health and the response to pain therapy. A pictogram (e.g., an emoji) can be embedded into the plot to enhance user perception of the feedback or the recommendation that the system provides to the user, such as a smiley face 732 for a high QoL score, or a frowning face 734 for a low QoL score, as illustrated in FIG. 7.

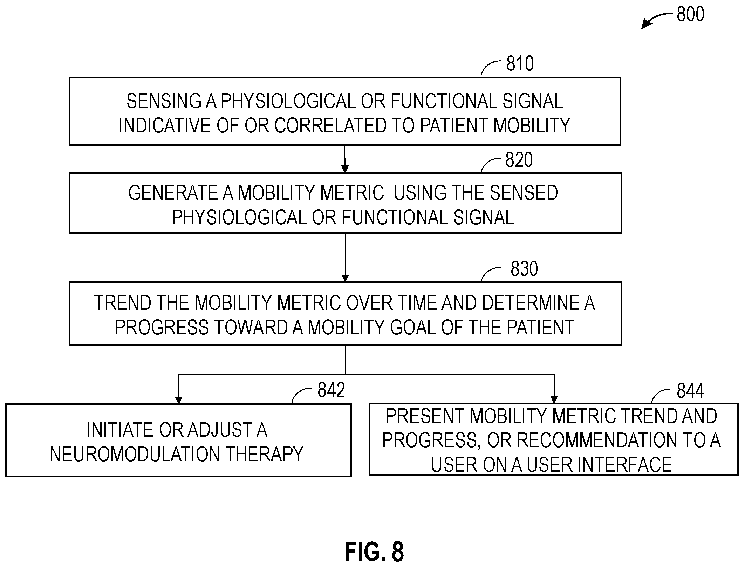

FIG. 8 illustrates, by way of example and not limitation, a non-claimed method 800 for monitoring and analyzing patient mobility, among other physiological and functional parameters, such as in the presence of pain or pain therapy (e.g., neuromodulation therapy). The method 800 can be implemented in and carried out by the mobility monitor system 300.

At 810, physiological or functional signals indicative of or correlated to patient mobility can be sensed from a patient using one or more implantable or external devices, such as the IPG 112, the wearable electronic device 120, or the mobile device 140. Examples of the received functional signals may include physical activity signals sensed by an activity sensor, such as an accelerometer (XL), a motor activity signal patient posture, gait, balance, or physical activity signals, a sleep state signal that contains information about sleep disturbance, among others. The functional signals may be collected by one or more sensors including, for example, an accelerometer, a gyroscope, a magnetometer (e.g., a compass), an inclinometer, a goniometers, an electromagnetic tracking system (ETS), or a global positioning system (GPS) sensor, among others. Examples of the received physiological signals can include electrocardiograms (ECGs), heart rate, heart rate variability, a photoplethysmography (PPG) signal, a blood oxygen saturation ($SpO_2$) signal, blood pressure, a respiration signal, respiratory rate, tidal volume, galvanic skin response, maximum rate of oxygen consumption ($VO2_{max}$) during exercise, peripheral body temperatures, a posture, or a sleep signal, among others. In some examples, patient reported outcome (PRO) may be received via a user interface of the wearable electronic device 120 or the mobile device 140. The PRO may include, for example, patient report on pain or paresthesia sensation (e.g., intensity, severity, duration, body location, or pattern of pain or paresthesia), effectiveness of pain therapy, patient report of activities, among others.

At 820, a mobility metric can be generated using the sensed physiological or function signal. The mobility metric may be represented by respective times spent in different activity intensity levels associated with one or more types of activities during a time period (e.g., a day, a number of days, a week, or other user-specified time period). The activity intensities may be categorized into one of a number of different mobility intensity bins, as illustrated in FIGS. 4A and 4B. In another example, the mobility metric may be represented a mobility score computed using a weighted combination of the intensity values each scaled by respective weight factors across all the intensity bins according to Equation (1), or using a weighted combination of the mobility times each scaled by respective weight factors across all the intensity bins according to Equation (2). In yet another example, the mobility metric may be computed using a dispersion metric, such as an entropy according to Equation (3), across the plurality of intensity bins.

The mobility metric may be generated using signals sensed by an accelerometer, such as a 3-axis accelerometer. Such mobility metrics are referred to as "standard" mobility metric. The mobility metric may alternatively be generated using one or more physiological or functional signals sensed by sensors other than an accelerometer. Such mobility metrics are referred to as "surrogate" mobility metric. One or more estimation models may be trained and calibrated against the accelerometer-based mobility metric values, such that the "surrogate" mobility metric estimated from various physiological or functional signals may be comparable to the standard, accelerometer-based mobility metric within a specified tolerance level (e.g., $\pm$ 5% or $\pm$ 10%).

In some examples, the mobility metric may include an activity-specific mobility metric that represents mobility times spent in a number of different activity intensities associated with a specific activity type or a specific activity context. Activity type or activity context, along with other activity information such as activity intensity and timing information (e.g., start time and end time), may be received from the user via a user interface. The user input, which can be in forms of textual or voice input, can be processed using a natural language processing (NLP) system to determine an association or a correlation between the description of the activity (e.g., words and or their frequencies) and the mobility metric (e.g., time spent at different activity intensities, or mobility scores). The physiological or functional signals acquired during the activity and the mobility metric generated therefrom can be "tagged" with the activity type or context provided by the patient.

In some examples, physiological or functional signals (and optionally the PRO information) can be collected in response to a neuromodulation therapy, such as SCS for controlling chronic pain. The neuromodulation therapy may be delivered in accordance with a stimulation setting, or one of a number of predetermined stimulation programs. Mobility metrics (e.g., mobility time distribution or mobility score) may be evaluated respectively for a number of different stimulation settings or stimulation programs. Mobility distributions can be generated respectively for different stimulation settings or programs over a predetermined time period, such as a week or a month in this example. Such stimulation setting or program-based mobility metric can improve interpretation of the impact of stimulation settings or programs on patient mobility, and enable more efficient individualized optimization of neuromodulation therapy. In some examples, the stimulation setting or program-based mobility metric may be evaluated for a specific activity type or activity context.

In some examples, the mobility metric determined at 820 can be compared to a population-based mobility metric to determine a relative mobility compared to other patients with similar medical conditions or similar demographics to the patient being evaluated. The population-based mobility metric can be generated using aggregated physiological or functional data collected from a patient group having a common characteristic as the patient, such as a patient group with chronic pain, a patient group with similar diagnosis of underly diseases, a patient group with similar baseline pain, a patient group with similar time of implant of implantable devices (e.g., IPG 112), a patient group with similar demographics (e.g., age) subgroup, or a patient group with similar comorbidities. The relative mobility may be represented by a percentile rank in the group of similarly patients. In an example, the relative mobility metric may be represented by a percentile of the population-based mobility metric for a user-specified patient group.

At 830, the mobility metric may be trended over time, and the patient progress toward a personalized mobility goal may be determined, such as using the trending circuit 353 and the progress assessment circuit 354, respectively. The mobility trend can be computed as a trend of mobility score or a trend of mobility time entropies over a number of days, weeks, or months. Additionally or alternatively, the mobility trend can be represented by a change in mobility distribution over time, including differences in mobility times associated with respective one or more activity intensity bins. The personalized mobility goal can be represented by a target mobility score, a target mobility time entropy, or a target mobility distribution. The personalized mobility goal can be determined based on a baseline mobility metric of the patient during a baseline time period when the patient performs a number of activities of different types and intensities. The baseline mobility metric represents a normal range of values of the mobility metric for the patient. The personalized mobility goal may additionally or alternatively be determined based on a population-based mobility metric generated from a group of patients having similar medical conditions or similar demographics to the patient being evaluated. In some examples, the personalized mobility goal can be determined or adjusted based on patient progress towards an existing mobility goal.

At 842, a neuromodulation therapy (e.g., SCS for chronic pain control) can be initiated or adjusted based on the trended mobility metric or the progress toward the personalized mobility goal. In an example, stimulation settings may be adjusted in accordance with the trended mobility metric or the progress toward the mobility goal. The neuromodulation therapy may be automatically delivered in accordance with the adjusted stimulation setting. Alternatively, the user can titrate the neuromodulation therapy in accordance with the trended mobility metric or the progress toward the mobility goal. In some examples, the personalized mobility goal can be adjusted based on patient progress towards an existing mobility goal. For example, if the patient hits the existing mobility goal, the mobility goal may be updated (e.g., increased by a specific factor, e.g., 1.25). If the patient is not progressing toward the existing goal as planned, the mobility goal may be reset (e.g., decrease by a specific factor, e.g., 1.25).

At 844, mobility information, including the trended mobility metric or the determined progress, can be presented to the user. This may include daily mobility distribution, or mobility distribution over a number of days or weeks or other specified time periods, and trends of mobility scores as shown in FIGS. 4A-4C, activity type- or activity context-based mobility metric as shown in FIG. 5A, mobility metrics for different stimulation settings or stimulation programs as shown in FIG. 5B, stimulation type and stimulation setting-based mobility metric as shown in FIG. 5C, changes from a previous mobility distribution (e.g., day-to-day different of mobility metric) as shown in FIG. 6A, or a comparison of the present-day mobility distribution to a target mobility distribution (as a personalized mobility goal) as shown in FIG. 6B. In some examples, a quality of life (QoL) score may be estimated based on the mobility metric and optionally the patient reported outcome (PRO), such as using the QoL estimator 357. The QoL may be presented to the user along with the trended mobility metric (e.g., a mobility score trend), as shown in FIG. 7. Additionally, a recommendation for future activities or a modification of the mobility goal (e.g., maintain or change of activity types, intensities, and respective durations at different activity intensities) may be presented to the user, such as via a user interface of the wearable electronic device 120, the mobile device 140, or the CP 228.

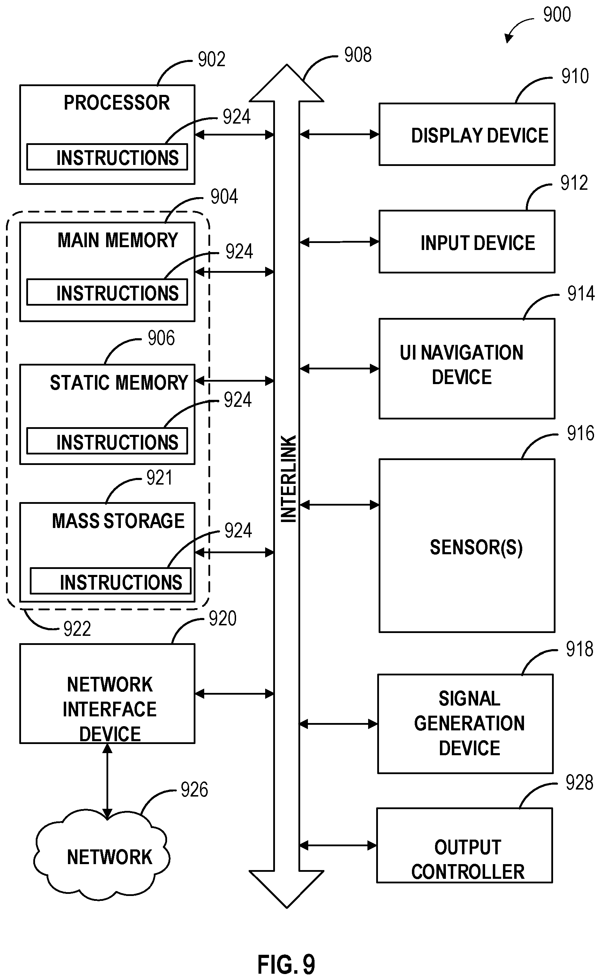

FIG. 9 illustrates generally a block diagram of an example machine 900 upon which any one or more of the techniques (e.g., methodologies) discussed herein may perform. Portions of this description may apply to the computing framework of various portions of the mobility monitor system 300, such as the controller circuit 350.

In alternative embodiments, the machine 900 may operate as a standalone device or may be connected (e.g., networked) to other machines. In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 900 may include a hardware processor 902 (e.g., a central processing unit (CPU), a graphics processing unit (GPU), a hardware processor core, or any combination thereof), a main memory 904 and a static memory 906, some or all of which may communicate with each other via an interlink (e.g., bus) 908. The machine 900 may further include a display unit 910 (e.g., a raster display, vector display, holographic display, etc.), an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensor. The machine 900 may include an output controller 928, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine readable medium 922 is illustrated as a single medium, the term "machine readable medium" may include a single medium or multiple media (e.g., a centralized or distributed database, and/or associated caches and servers) configured to store the one or more instructions 924.

The term "machine readable medium" may include any medium that is capable of storing, encoding, or carrying instructions for execution by the machine 900 and that cause the machine 900 to perform any one or more of the techniques of the present disclosure, or that is capable of storing, encoding or carrying data structures used by or associated with such instructions. Nonlimiting machine readable medium examples may include solid-state memories, and optical and magnetic media. In an example, a massed machine readable medium comprises a machine readable medium with a plurality of particles having invariant (e.g., rest) mass. Accordingly, massed machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 924 may further be transmitted or received over a communications network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi®, IEEE 802.16 family of standards known as WiMax®), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communications network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 900, and includes digital or analog communications signals or other intangible medium to facilitate communication of such software.

Various embodiments are illustrated in the figures above. One or more features from one or more of these embodiments may be combined to form other embodiments.

The non-claimed method examples described herein can be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code can form portions of computer program products. Further, the code can be tangibly stored on one or more volatile or non-volatile computer-readable media during execution or at other times.

The above detailed description is intended to be illustrative, and not restrictive. The scope of the disclosure should, therefore, be determined with references to the appended claims.

**Claims**

1. A system (100; 300) for monitoring mobility of a patient treated with a neuromodulation therapy, the system comprising:

   a sensor circuit configured to sense a physiological or functional signal indicative of or correlated to patient mobility;
   an electrostimulator (360) configured to generate and deliver the neuromodulation therapy to the patient; and
   a controller circuit (350) configured to:

   generate a mobility metric using the sensed physiological or functional signal, the mobility metric representing mobility times spent in respective different activity intensities associated with one or more types of activities during a specific time period;
   trend the mobility metric over time and determine a progress toward a mobility goal of the patient; and
   generate a control signal to the electrostimulator (360) to initiate or adjust the neuromodulation therapy in accordance with the trended mobility metric or the determined progress toward the mobility goal.

2. The system of claim 1, wherein the controller circuit (350) is configured to:

   based on the trended mobility metric or the determined progress toward the mobility goal, generate a recommendation for future activities or a modification of the mobility goal; and

present the recommendation, and the trended mobility metric or the determined progress, on a user interface.

3. The system of any of claims 1-2, wherein the mobility metric includes an activity-specific mobility metric representing mobility times spent in the respective different activity intensities associated with a specific activity type or a specific activity context,
wherein the controller circuit (350) is further configured to:

trend the activity-specific mobility metric over time and determine a progress toward an activity-specific mobility goal of the patient; and
generate the control signal to the electrostimulator (360) to initiate or adjust the neuromodulation therapy in accordance with the trended activity-specific mobility metric or the determined progress toward the activity-specific mobility goal when the patient engages in an activity of the specific activity type or under the specific activity context.

4. The system of claim 3, wherein the controller circuit (350) is further configured to:

generate respective activity-specific mobility metrics for one or more activity types or activity contexts; and
present on a user interface an association between (i) the one or more activity types or activity contexts and (ii) the respective activity-specific mobility metrics.

5. The system of claim 4, wherein the controller circuit (350) is configured to:

receive from the user interface a user input of the one or more activity types or activity contexts during the specific time period; and
correlate the user input to the respective activity-specific mobility metrics.

6. The system of any of claims 1-5, wherein the controller circuit (350) is further configured to:

generate (i) a first mobility metric from a first physiological or functional signal sensed in response to neurostimulation according to a first stimulation program, and (ii) a second mobility metric from a second physiological or functional signal sensed in response to neurostimulation according to a second stimulation program different than the first stimulation program;
trend respectively the first mobility metric and the second mobility metric over time, and determine respective progresses toward the mobility goal of the patient; and
generate the control signal to the electrostimulator (360) to initiate or adjust the neuromodulation therapy using one of the first or the second stimulation program selected based on a comparison of the trended first mobility metric to the trended second mobility metric, or a comparison between the respective progresses toward the mobility goal.

7. The system of claim 6, wherein the first and the second physiological or functional signals are sensed when the patient engages in a same type of activity or under a same activity context,
wherein the controller circuit (350) is configured to generate the control signal to the electrostimulator (360) to initiate or adjust the neuromodulation therapy using the selected stimulation program when the patient engages in the same type of activity or under the same activity context.

8. The system of any of claims 1-7, wherein the controller circuit (350) is configured to categorize the different activity intensities into a plurality of intensity bins, and to generate the mobility metric including an entropy of the mobility times across the plurality of intensity bins.

9. The system of any of claims 1-8, wherein:

the sensor circuit includes a physiological sensor configured to sense a physiological signal correlated to patient mobility; and
the controller circuit (350) is configured to apply the sensed physiological signal to a trained estimation model to generate the mobility metric.

10. The system of claim 9, comprising a model training circuit configured to:

receive a training dataset comprising a set of activity signals sensed by accelerometers and a set of physiological signals sensed by physiological sensors different from the accelerometers, the set of activity signals and the set of physiological signals sensed substantially concurrently from the patient;

determine accelerometer-based mobility metric values using the set of activity signals; and

generate the trained estimation model using the training dataset and the accelerometer-based mobility metric values, the trained estimation model mapping the set of physiological signals to the accelerometer-based mobility metric values.

11. The system of any of claims 1-10, wherein the controller circuit (350) is configured to:

generate a population-based mobility metric using physiological or functional signals sensed from a number of patients having similar medical conditions or similar demographics to the patient; and

determine, and present on a user interface, a relative position of the mobility metric of the patient with respect to the population-based mobility metric.

12. The system of any of claims 1-11, wherein the controller circuit (350) is configured to determine the mobility goal based on at least one of:

a baseline mobility metric of the patient during a baseline time period; or

a population-based mobility metric generated from patients having similar medical conditions or similar demographics to the patient.

13. The system of any of claims 1-12, wherein the controller circuit (350) is configured to:

categorize the different activity intensities into a plurality of intensity bins; and

present, on a user interface, a graphical comparison between (i) the mobility metric represented by a distribution of the mobility times across the plurality of intensity bins and (ii) the mobility goal represented by a target distribution of target mobility times across the plurality of intensity bins.

14. The system of any of claims 1-13, wherein the controller circuit (350) is configured to:

categorize the different activity intensities into a plurality of intensity bins; and

present, on a user interface, a graphical comparison between (i) a first distribution of mobility times across the plurality of intensity bins during a first time period and (ii) a second distribution of mobility times across the plurality of intensity bins during a second time period prior to the first time period.

15. The system of any of claims 1-14, wherein the controller circuit (350) is configured to:

establish a correlation between the mobility metric and a patient-reported functional state of the patient; and

predict a future functional state of the patient using the established correlation.

**Patentansprüche**

1. System (100; 300) zur Überwachung der Mobilität eines Patienten, der mit einer Neuromodulationstherapie behandelt wird, wobei das System aufweist:

eine Sensorschaltung, die zum Erfassen eines physiologischen oder funktionalen Signals, das die Mobilität des Patienten indiziert oder mit ihr korreliert, konfiguriert ist;

einen Elektrostimulator (360), der zum Erzeugen der Neuromodulationstherapie und zu deren Abgabe an den Patienten konfiguriert ist; und

eine Steuerschaltung (350), die konfiguriert ist:

eine Mobilitätsmetrik unter Verwendung des erfassten physiologischen oder funktionalen Signals zu erzeugen, wobei die Mobilitätsmetrik die Mobilitätszeiten darstellt, die in den jeweiligen unterschiedlichen Aktivitätsintensitäten verbracht wurden, die mit einer oder mehreren Arten von Aktivitäten während einer bestimmten Zeitspanne verbunden sind;

die Mobilitätsmetrik im Zeitverlauf trendmäßig auszuwerten und einen Fortschritt in Richtung eines Mobilitätsziels des Patienten zu bestimmen; und

ein Steuersignal für den Elektrostimulator (360) zu erzeugen, um die Neuromodulationstherapie in Übereinstimmung mit dem ermittelten Trend der Mobilitätsmetrik oder dem bestimmten Fortschritt in Richtung des Mobilitätsziels einzuleiten oder anzupassen.

2. System nach Anspruch 1, wobei die Steuerschaltung (350) konfiguriert ist zum:

Erzeugen einer Empfehlung für künftige Aktivitäten oder eine Änderung des Mobilitätsziels auf der Grundlage der trendmäßig ausgewerteten Mobilitätsmetrik oder des bestimmten Fortschritts in Richtung des Mobilitätsziels; und

Darstellen der Empfehlung und der trendmäßig ausgewerteten Mobilitätsmetrik oder des bestimmten Fortschritts auf einer Benutzeroberfläche.

3. System nach einem der Ansprüche 1-2, wobei die Mobilitätsmetrik eine aktivitätsspezifische Mobilitätsmetrik aufweist, die Mobilitätszeiten darstellt, die in den jeweiligen unterschiedlichen Aktivitätsintensitäten verbracht werden, die mit einem spezifischen Aktivitätstyp oder einem spezifischen Aktivitätskontext verbunden sind, wobei die Steuerschaltung(350) ferner konfiguriert ist:

die aktivitätsspezifische Mobilitätsmetrik im Zeitverlauf trendmäßig auszuwerten und einen Fortschritt in Richtung eines aktivitätsspezifischen Mobilitätsziels des Patienten zu bestimmen; und

das Steuersignal für den Elektrostimulator (360) zu erzeugen, um die Neuromodulationstherapie in Übereinstimmung mit der trendmäßig ausgewerteten aktivitätsspezifischen Mobilitätsmetrik oder dem bestimmten Fortschritt in Richtung des aktivitätsspezifischen Mobilitätsziels einzuleiten oder anzupassen, wenn der Patient eine Aktivität des spezifischen Aktivitätstyps oder in dem spezifischen Aktivitätskontext ausübt.

4. System nach Anspruch 3, wobei die Steuerschaltung (350) ferner konfiguriert ist zum:

Erzeugen von jeweiligen aktivitätsspezifischen Mobilitätsmetriken für eine oder mehrere Aktivitätsarten oder Aktivitätskontexte; und

Darstellen einer Zuordnung zwischen (i) dem einen oder den mehreren Aktivitätstypen oder Aktivitätskontexten und (ii) den jeweiligen aktivitätsspezifischen Mobilitätsmetriken auf einer Benutzeroberfläche.

5. System nach Anspruch 4, wobei die Steuerschaltung (350) konfiguriert ist zum:

Empfangen einer Benutzereingabe des einen oder der mehreren Aktivitätstypen oder Aktivitätskontexte während des spezifischen Zeitraums von der Benutzeroberfläche; und

Korrelieren der Benutzereingaben mit den jeweiligen aktivitätsspezifischen Mobilitätsmetriken.

6. System nach Anspruch einem der Ansprüche 1-5, wobei die Steuerschaltung (350) ferner konfiguriert ist zum:

Erzeugen (i) einer ersten Mobilitätsmetrik aus einem ersten physiologischen oder funktionalen Signal, das als Reaktion auf Neurostimulation gemäß einem ersten Stimulationsprogramm erfasst wird, und (ii) einer zweiten Mobilitätsmetrik aus einem zweiten physiologischen oder funktionalen Signal, das als Reaktion auf Neurostimulation gemäß einem zweiten, von dem ersten Stimulationsprogramm verschiedenen Stimulationsprogramm erfasst wird;

trendmäßigen Auswerten der ersten Mobilitätsmetrik und der zweiten Mobilitätsmetrik im Zeitverlauf und Bestimmen der jeweiligen Fortschritte in Richtung des Mobilitätsziels des Patienten; und

Erzeugen des Steuersignals für den Elektrostimulator (360), um die Neuromodulationstherapie unter Verwendung des ersten oder des zweiten Stimulationsprogramms, das auf der Grundlage eines Vergleichs der trendmäßig ausgewerteten ersten Mobilitätsmetrik mit der trendmäßig ausgewerteten zweiten Mobilitätsmetrik oder eines Vergleichs zwischen den jeweiligen Fortschritten in Richtung des Mobilitätsziels ausgewählt wurde, einzuleiten oder anzupassen.

7. System nach Anspruch 6, wobei das erste und das zweite physiologische oder funktionale Signal erfasst werden, wenn der Patient die gleiche Art von Aktivität ausübt oder sich in dem gleichen Aktivitätskontext befindet, wobei die Steuerschaltung (350) zum Erzeugen des Steuersignals für den Elektrostimulator (360) konfiguriert ist, um die Neuromodulationstherapie unter Verwendung des ausgewählten Stimulationsprogramms einzuleiten oder anzupassen, wenn der Patient die gleiche Art von Aktivität oder im gleichen Aktivitätskontext ausübt.

8. System nach einem der Ansprüche 1-7, wobei die Steuerschaltung (350) zum Kategorisieren der verschiedenen Aktivitätsintensitäten in mehrere Intensitätsbins und zum Erzeugen der Mobilitätsmetrik einschließlich einer Entropie der Mobilitätszeiten über die mehreren Intensitätsbins hinweg konfiguriert ist.

9. System nach einem der Ansprüche 1-8, wobei:

die Sensorschaltung einen physiologischen Sensor aufweist, der zum Erfassen eines mit der Mobilität des Patienten korrelierenden physiologischen Signals konfiguriert ist; und
die Steuerschaltung (350) so zum Anwenden des erfassten physiologischen Signals auf ein trainiertes Schätzungsmodell konfiguriert ist, um die Mobilitätsmetrik zu erzeugen.

10. System nach Anspruch 9, aufweisend eine Modelltrainingsschaltung, die konfiguriert ist zum:

Empfangen eines Trainingsdatensatzes, der einen Satz von Aktivitätssignalen, die von Beschleunigungsmessern erfasst werden, und einen Satz von physiologischen Signalen, die von physiologischen Sensoren erfasst werden, die sich von den Beschleunigungsmessern unterscheiden, aufweist, wobei der Satz von Aktivitätssignalen und der Satz von physiologischen Signalen von dem Patient im Wesentlichen gleichzeitig erfasst werden;
Bestimmen von beschleunigungsmesserbasierten Mobilitätsmetrikwerten unter Verwendung des Satzes von Aktivitätssignalen; und
Erzeugen des trainierten Schätzmodells unter Verwendung des Trainingsdatensatzes und der beschleunigungsmesserbasierten Mobilitätsmesswerte, wobei das trainierte Schätzmodell den Satz physiologischer Signale auf die beschleunigungsmesserbasierten Mobilitätsmesswerte abbildet.

11. System nach einem der Ansprüche 1-10, wobei die Steuerschaltung (350) konfiguriert ist zum:

Erzeugen einer populationsbasierten Mobilitätsmetrik unter Verwendung physiologischer oder funktionaler Signale, die von einer Anzahl von Patienten mit ähnlichen Erkrankungen oder ähnlichen demografischen Merkmalen wie der Patient erfasst werden; und
Bestimmen einer relativen Position der Mobilitätsmetrik des Patienten in Bezug auf die populationsbasierte Mobilitätsmetrik und Darstellen derselben auf einer Benutzeroberfläche.

12. System nach einem der Ansprüche 1-11, wobei die Steuerschaltung (350) zum Bestimmen des Mobilitätsziels auf der Grundlage von mindestens einem der folgenden konfiguriert ist:

eine Ausgangsmobilitätsmetrik des Patienten während einer Ausgangszeitspanne; oder
eine populationsbezogene Mobilitätsmetrik, die von Patienten mit ähnlichen Erkrankungen oder ähnlichen demografischen Merkmalen wie der Patient erstellt wird.

13. System nach einem der Ansprüche 1-12, wobei die Steuerschaltung (350) konfiguriert ist zum:

Kategorisieren der verschiedenen Aktivitätsintensitäten in mehrere Intensitätsbins; und
Darstellen eines grafischen Vergleichs zwischen (i) der Mobilitätsmetrik, die durch eine Verteilung der Mobilitätszeiten über die mehreren Intensitätsbins dargestellt wird, und (ii) dem Mobilitätsziel, das durch eine Zielverteilung der Zielmobilitätszeiten über die mehreren Intensitätsbins dargestellt wird, auf einer Benutzeroberfläche.

14. System nach einem der Ansprüche 1-13, wobei die Steuerschaltung (350) konfiguriert ist zum:

Kategorisieren der verschiedenen Aktivitätsintensitäten in mehrere Intensitätsbins; und
Darstellen eines grafischen Vergleichs zwischen (i) einer ersten Verteilung von Mobilitätszeiten über die mehreren Intensitätsbins während einer ersten Zeitspanne und (ii) einer zweiten Verteilung von Mobilitätszeiten über die mehreren Intensitätsbins während einer zweiten Zeitspanne vor der ersten Zeitspanne auf einer Benutzeroberfläche.

15. System nach einem der Ansprüche 1-14, wobei die Steuerschaltung (350) konfiguriert ist zum:

Herstellen einer Korrelation zwischen der Mobilitätsmetrik und einem vom Patienten angegebenen Funktionszustand des Patienten; und

Vorhersagen eines zukünftigen Funktionszustands des Patienten anhand der hergestellten Korrelation.

**Revendications**

1. Système (100 ; 300) de surveillance de la mobilité d'un patient traité par une thérapie de neuromodulation, le système comprenant :

   un circuit de capteur configuré pour détecter un signal physiologique ou fonctionnel indicatif de la mobilité du patient ou corrélé à celle-ci ;
   un électrostimulateur (360) configuré pour générer et délivrer la thérapie de neuromodulation au patient; et
   un circuit de contrôleur (350) configuré pour :

   générer une métrique de mobilité à l'aide du signal physiologique ou fonctionnel détecté, la métrique de mobilité représentant les temps de mobilité passés dans différentes intensités d'activité respectives associées à un ou plusieurs types d'activités pendant une période de temps spécifique ;
   suivre la tendance de la métrique de mobilité au fil du temps et déterminer un progrès accompli vers un objectif de mobilité du patient ; et
   générer un signal de commande au niveau de l'électrostimulateur (360) afin d'initier ou d'ajuster la thérapie de neuromodulation conformément à la métrique de mobilité dont la tendance est suivie ou au progrès déterminé vers l'objectif de mobilité.

2. Système selon la revendication 1, dans lequel le circuit de contrôleur (350) est configuré pour :

   sur la base de la métrique de mobilité dont la tendance est suivie ou du progrès déterminé vers l'objectif de mobilité, générer une recommandation pour des activités futures ou une modification de l'objectif de mobilité ; et
   présenter la recommandation, et la métrique de mobilité dont la tendance est suivie ou le progrès déterminé, sur une interface utilisateur.

3. Système selon l'une quelconque des revendications 1 à 2, dans lequel la métrique de mobilité comprend une métrique de mobilité spécifique à l'activité représentant les temps de mobilité passés dans les différentes intensités d'activité respectives associées à un type d'activité spécifique ou à un contexte d'activité spécifique,
   dans lequel le circuit de contrôleur (350) est en outre configuré pour :

   suivre la tendance de la métrique de mobilité spécifique à l'activité au fil du temps, et déterminer un progrès accompli vers un objectif de mobilité spécifique à l'activité du patient; et
   générer le signal de commande au niveau de l'électrostimulateur (360) pour initier ou ajuster la thérapie de neuromodulation conformément à la métrique de mobilité spécifique à l'activité dont la tendance est suivie, ou au progrès déterminé vers l'objectif de mobilité spécifique à l'activité lorsque le patient s'engage dans une activité du type d'activité spécifique, ou dans le contexte d'activité spécifique.

4. Système selon la revendication 3, dans lequel le circuit de contrôleur (350) est en outre configuré pour :

   générer des métriques de mobilité respectives spécifiques à une activité pour un ou plusieurs types d'activité ou contextes d'activité ; et
   présenter, sur une interface utilisateur, une association entre (i) les un ou plusieurs types d'activité ou contextes d'activité et (ii) les métriques de mobilité respectives spécifiques à l'activité.

5. Système selon la revendication 4, dans lequel le circuit de contrôleur (350) est configuré pour :

   recevoir, en provenance de l'interface utilisateur, une entrée d'utilisateur des un ou plusieurs types d'activité ou contextes d'activité pendant la période de temps spécifique ; et
   corréler l'entrée d'utilisateur aux métriques de mobilité respectives spécifiques à l'activité.

6. Système selon l'une quelconque des revendications 1 à 5, dans lequel le circuit de contrôleur (350) est en outre configuré pour :

générer (i) une première métrique de mobilité à partir d'un premier signal physiologique ou fonctionnel détecté en réponse à une neurostimulation selon un premier programme de stimulation, et (ii) une deuxième métrique de mobilité à partir d'un deuxième signal physiologique ou fonctionnel détecté en réponse à une neurostimulation selon un deuxième programme de stimulation, différent du premier programme de stimulation ;

suivre respectivement la tendance de la première métrique de mobilité et de la deuxième métrique de mobilité au fil du temps, et déterminer les progrès respectifs accomplis vers l'objectif de mobilité du patient; et

générer le signal de commande au niveau de l'électrostimulateur (360) pour initier ou ajuster la thérapie de neuromodulation en utilisant l'un du premier ou du deuxième programme de stimulation sélectionné, sur la base d'une comparaison entre la première métrique de mobilité dont la tendance est suivie et la deuxième métrique de mobilité dont la tendance est suivie, ou d'une comparaison entre les progrès respectifs vers l'objectif de mobilité.

7. Système selon la revendication 6, dans lequel le premier et le deuxième signaux physiologiques ou fonctionnels sont détectés lorsque le patient s'engage dans un même type d'activité, ou dans un même contexte d'activité,

dans lequel le circuit de contrôleur (350) est configuré pour générer le signal de commande au niveau de l'électrostimulateur (360) afin d'initier ou d'ajuster la thérapie de neuromodulation à l'aide du programme de stimulation sélectionné lorsque le patient s'engage dans le même type d'activité, ou dans le même contexte d'activité.

8. Système selon l'une quelconque des revendications 1 à 7, dans lequel le circuit de contrôleur (350) est configuré pour catégoriser les différentes intensités d'activité dans une pluralité de classes d'intensité, et pour générer la métrique de mobilité comprenant une entropie des temps de mobilité à travers la pluralité de classes d'intensité.

9. Système selon l'une quelconque des revendications 1 à 8, dans lequel :

le circuit de capteur comprend un capteur physiologique configuré pour détecter un signal physiologique corrélé à une mobilité de patient; et

le circuit de contrôleur (350) est configuré pour appliquer le signal physiologique détecté à un modèle d'estimation entraîné, afin de générer la métrique de mobilité.

10. Système selon la revendication 9, comprenant un circuit d'entraînement de modèle configuré pour :

recevoir un ensemble de données d'entraînement comprenant un ensemble de signaux d'activité détectés par des accéléromètres et un ensemble de signaux physiologiques détectés par des capteurs physiologiques différents des accéléromètres, l'ensemble de signaux d'activité et l'ensemble de signaux physiologiques étant détectés sensiblement simultanément à partir du patient ;

déterminer des valeurs de métrique de mobilité basées sur les accéléromètres à l'aide de l'ensemble de signaux d'activité ; et

générer le modèle d'estimation entraîné à l'aide de l'ensemble de données d'entraînement et des valeurs de métrique de mobilité basées sur les accéléromètres, le modèle d'estimation entraîné mettant en correspondance l'ensemble de signaux physiologiques avec les valeurs de métrique de mobilité basées sur les accéléromètres.

11. Système selon l'une quelconque des revendications 1 à 10, dans lequel le circuit de contrôleur (350) est configuré pour :

générer une métrique de mobilité basée sur une population à l'aide des signaux physiologiques ou fonctionnels détectés à partir d'un nombre de patients ayant des conditions médicales similaires ou des caractéristiques démographiques similaires à celles du patient ; et

déterminer, et présenter sur une interface utilisateur, une position relative de la métrique de mobilité du patient par rapport à la métrique de mobilité basée sur la population.

12. Système selon l'une quelconque des revendications 1 à 11, dans lequel le circuit de contrôleur (350) est configuré pour déterminer l'objectif de mobilité sur la base d'au moins l'une des métriques suivantes :

une métrique de mobilité de référence du patient pendant une période de temps de référence ; ou

une métrique de mobilité basée sur la population, générée à partir de patients ayant des conditions médicales similaires ou des caractéristiques démographiques similaires à celles du patient.

13. Système selon l'une quelconque des revendications 1 à 12, dans lequel le circuit de contrôleur (350) est configuré pour :

catégoriser les différentes intensités d'activité dans une pluralité de classes d'intensité ; et

présenter, sur une interface utilisateur, une comparaison graphique entre (i) la métrique de mobilité représentée par une distribution des temps de mobilité dans la pluralité de classes d'intensité et (ii) l'objectif de mobilité représenté par une distribution cible des temps de mobilité cibles dans la pluralité de classes d'intensité.

14. Système selon l'une quelconque des revendications 1 à 13, dans lequel le circuit de contrôleur (350) est configuré pour :

catégoriser les différentes intensités d'activité dans une pluralité de classes d'intensité ; et

présenter, sur une interface utilisateur, une comparaison graphique entre (i) une première distribution des temps de mobilité dans la pluralité de classes d'intensité pendant une première période de temps et (ii) une deuxième distribution des temps de mobilité dans la pluralité de classes d'intensité pendant une deuxième période de temps précédant la première période de temps.

15. Système selon l'une quelconque des revendications 1 à 14, dans lequel le circuit de contrôleur (350) est configuré pour :

établir une corrélation entre la métrique de mobilité et un état fonctionnel du patient qui est signalé par le patient ; et

prédire un état fonctionnel futur du patient à l'aide de la corrélation établie.

FIG. 1

**FIG. 2**

**FIG. 3**

410

**Daily Mobility Time Distribution**

Watching TV, Having Dinner

Grocery Shopping, Walking my Dog

Yardwork

Jogging

Gym Workout

Time

Bin 1   Bin 2   Bin 3   Bin 4   Bin 5   Bin 6

**Bin 1-5: Intensity Bins (Ascending Intensities): Rest, Low, Low Med, Med-Hi, and Hi**

**FIG. 4A**

420

**Mobility Times Over Days**

☐ Rest    ⊠ Med
⊠ Low     ⊠ Med_high
⊠ Low_med ⊠ High

Hours

20

15

10

5

0

8/01    8/08    8/15    8/22

**FIG. 4B**

430

## Mobility Scores Over Days

## FIG. 4C

## Activity Type or Context-Based Mobility Metric

| Activity Types / Contexts | Mobility Distribution | Mobility Score (S) |
|---|---|---|
| Grocery Shopping | | 0.2 |
| Walking my Dog | | 0.4 |
| Gym Workout | | 0.9 |

## FIG. 5A

## Therapy-Based Mobility Metric

| Stimulation Settings/Programs | Mobility Distribution | Mobility Score (S) |
|---|---|---|
| Stimulation Setting A | | 0.2 |
| Stimulation Setting B | | 0.6 |
| Stimulation Setting C | | 0.8 |

**FIG. 5B**

## Activity Type/Context and Therapy-Based Mobility Metric

| Stimulation Settings/Programs | Mobility Distribution | Mobility Score (S) |
|---|---|---|
| Stimulation setting A, during "walking my dog" | | 0.5 |
| Stimulation setting B, during "walking my dog" | | 0.7 |
| Stimulation setting C, during "walking my dog" | | 0.9 |

**FIG. 5C**

**Change of Mobility Distribution**

FIG. 6A

**Current vs. Target Mobility Distribution**

FIG. 6B

FIG. 7

EP 4 572 839 B1

```
                                              ┌─ 800
                                        ┌─ 810
┌──────────────────────────────────────────────────────────┐
│  SENSING A PHYSIOLOGICAL OR FUNCTIONAL SIGNAL            │
│  INDICATIVE OF OR CORRELATED TO PATIENT MOBILITY        │
└──────────────────────────────────────────────────────────┘
                          │               ┌─ 820
                          ▼
┌──────────────────────────────────────────────────────────┐
│  GENERATE A MOBILITY METRIC  USING THE SENSED           │
│  PHYSIOLOGICAL OR FUNCTIONAL SIGNAL                      │
└──────────────────────────────────────────────────────────┘
                          │               ┌─ 830
                          ▼
┌──────────────────────────────────────────────────────────┐
│  TREND THE MOBILITY METRIC OVER TIME AND DETERMINE A     │
│  PROGRESS TOWARD A MOBILITY GOAL OF THE PATIENT          │
└──────────────────────────────────────────────────────────┘
              │                                    │
    ┌─ 842    ▼                          ┌─ 844    ▼
┌──────────────────────────┐      ┌──────────────────────────────┐
│  INITIATE OR ADJUST A    │      │  PRESENT MOBILITY METRIC     │
│  NEUROMODULATION THERAPY │      │  TREND AND PROGRESS, OR      │
│                          │      │  RECOMMENDATION TO A         │
│                          │      │  USER ON A USER INTERFACE    │
└──────────────────────────┘      └──────────────────────────────┘
```

**FIG. 8**

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10631777 B2
- US 8019439 B
- US 7650184 B